# EUROPEAN PATENT SPECIFICATION

(11) **EP 1 733 041 B1**
(45) Date of publication and mention of the grant of the patent: **09.05.2012**
(21) Application number: 05734951.6
(22) Date of filing: 17.03.2005
(51) Int. Cl.: C07K 16/22, G01N 33/53, C07K 16/00, A61K 39/40, A61K 39/42, A61K 39/395, C12P 21/08

(54) **ANTI-MYOSTATIN ANTIBODIES**
ANTI-MYOSTATIN-ANTIKÖRPER
ANTICORPS DIRIGES CONTRE LA MYOSTATINE

(30) Priority: 23.03.2004 US 555456 P; 05.04.2004 US 559621 P
(43) Date of publication of application: 20.12.2006
(73) Proprietor: Eli Lilly & Company, Indianapolis, IN 46285 (US)
(72) Inventor: SMITH, Rosamund, Carol, Greenfield, IN 46140 (US); KIKLY, Kristine, Kay, Fortville, IN 46040 (US); TOBIAS, Linda, O., Indianapolis, IN 46208 (US); HAN, Bomie, Carmel, IN 46033 (US)
(74) Representative: Kent, Lindsey Ruth
(86) International application number: PCT/US2005/009307
(87) International publication number: WO 2005/094446

(56) References cited:
- US-A- 6 018 032
- US-A- 6 096 506
- US-A- 6 103 466
- US-A1- 2003 138 422
- HOLT L J ET AL: "Domain antibodies: proteins for therapy" TRENDS IN BIOTECHNOLOGY, ELSEVIER PUBLICATIONS, CAMBRIDGE, GB, vol. 21, no. 11, 1 November 2003 (2003-11-01), pages 484-490, XP004467495 ISSN: 0167-7799
- DAVIES J ET AL: "Affinity improvement of single antibody VH domains: residues in all three hypervariable regions affect antigen binding" IMMUNOTECHNOLOGY, ELSEVIER SCIENCE PUBLISHERS BV, NL, vol. 2, no. 3, 1 September 1996 (1996-09-01), pages 169-179, XP004070292 ISSN: 1380-2933
- LITTLE M ET AL: "Of mice and men: hybridoma and recombinant antibodies" IMMUNOLOGY TODAY, ELSEVIER PUBLICATIONS, CAMBRIDGE, GB, vol. 21, no. 8, 1 August 2000 (2000-08-01), pages 364-370, XP004215163 ISSN: 0167-5699
- MCPHERRON A C ET AL: "REGULATION OF SKELETAL MUSCLE MASS IN MICE BY A NEW TGF-BETA SUPERFAMILY MEMBER" NATURE, NATURE PUBLISHING GROUP, LONDON, UK, vol. 387, 1 May 1997 (1997-05-01), pages 83-90, XP002936996 ISSN: 0028-0836
- TESSARI PAOLO: "Protein metabolism in liver cirrhosis: from albumin to muscle myofibrils.", CURRENT OPINION IN CLINICAL NUTRITION AND METABOLIC CARE JAN 2003 LNKD- PUBMED:12496684, vol. 6, no. 1, January 2003 (2003-01), pages 79-85, ISSN: 1363-1950
- PASINI EVASIO ET AL: "Malnutrition, muscle wasting and cachexia in chronic heart failure: the nutritional approach.", ITALIAN HEART JOURNAL : OFFICIAL JOURNAL OF THE ITALIAN FEDERATION OF CARDIOLOGY APR 2003 LNKD- PUBMED:12784775, vol. 4, no. 4, April 2003 (2003-04), pages 232-235, ISSN: 1129-471X
- KAIZU YUKIKO ET AL: "Association between inflammatory mediators and muscle mass in long-term hemodialysis patients.", AMERICAN JOURNAL OF KIDNEY DISEASES : THE OFFICIAL JOURNAL OF THE NATIONAL KIDNEY FOUNDATION AUG 2003 LNKD- PUBMED:12900811, vol. 42, no. 2, August 2003 (2003-08), pages 295-302, ISSN: 1523-6838
- MCPHERRON ALEXANDRA C ET AL: "Suppression of body fat accumulation in myostatin-deficient mice.", THE JOURNAL OF CLINICAL INVESTIGATION MAR 2002 LNKD- PUBMED:11877467, vol. 109, no. 5, March 2002 (2002-03), pages 595-601, ISSN: 0021-9738
- WOUTERS E F M: "Chronic obstructive pulmonary disease. 5: systemic effects of COPD.", THORAX DEC 2002 LNKD- PUBMED:12454303, vol. 57, no. 12, December 2002 (2002-12), pages 1067-1070, ISSN: 0040-6376

## Description

### FIELD OF THE INVENTION

The present invention is in the field of medicine, particularly in the field of monoclonal antibodies against myostatin. More specifically the invention relates to neutralizing anti-myostatin monoclonal antibodies that bind a novel epitope identified on the mature form of myostatin. The antibodies of the invention may be murine, chimeric, or humanized antibodies, immunoconjugates of the antibodies or antigen-binding fragments thereof. The antibodies of the invention are useful in mammals for increasing muscle mass, increasing bone density, or for the treatment of conditions wherein the presence of myostatin causes or contributes to undesirable pathological effects or wherein a decrease in myostatin levels contributes to a desirable therapeutic effect.

### BACKGROUND OF THE INVENTION

Members of the transforming growth factor beta (TGF-β) superfamily of proteins are involved in embryonic development and adult tissue homeostasis. The TGF-β superfamily members share a common structure including a short peptide signal sequence required for secretion of the protein and an amino-terminal fragment that is proteolytically cleaved about 105 - 140 amino acids from the carboxy-terminus of the large precursor protein ("proprotein") to produce the mature protein. The mature protein is characterized by highly conserved cysteine residues, while the active form of the protein is a disulfide-linked dimer of the mature protein (Gray, A., and Maston, A., Science, 247:1328, 1990). Heterodimers of members of the TGF-β superfamily have also been detected and appear to have different biological properties than the homodimers.

Myostatin, also referred to as growth differentiation factor-8 (GDF-8) is a member of the TGF-β superfamily of proteins. Myostatin is expressed primarily in developing and adult skeletal muscle and functions as a negative regulator of skeletal muscle. Myostatin is highly conserved across species; the amino acid sequence of the mature form of myostatin in human, mouse, rat and cow are 100% identical. The immunogenic epitope identified in the present invention is 100% identical in human, mouse, rat, chicken, dog, horse, goat, sheep, cow and pig. Growth differentiation factor-11, also referred to as GDF-11 or BMP-11, is the member of the TGF-β superfamily of proteins that is most homologous to myostatin. Human myostatin and GDF-11 are 90% identical on the amino acid level within their mature chain.

U.S. Patent Number 5,827,733 teaches the polynucleotide sequence and amino acid sequence of human myostatin while U.S. Patent Number 6,096,506 claims an antibody specifically reactive with GDF-8 polypeptide or an epitope thereof. U.S. Patent Application 2003/0138422 claims an antibody that specifically binds a GDF-8 protein comprising a particular peptide. U.S. Patent Number 6,468,535 claims a method for increasing animal muscle mass by administration of an anti-GDF-8 antibody. U.S. Patent Number 6,368,597 teaches using a GDF-8 antibody for treating diabetes.

There are presently limited effective treatments for disorders or conditions which would benefit from an increase in muscle mass and/or muscle strength including muscular dystrophy, frailty, critical care myopathy, and cachexia resulting from cancer or other disorders, including but not limited to HIV infection, critical care and myopathies. Due to its role as a negative regulator of skeletal muscle growth, myostatin is a desirable target for therapeutic intervention for such disorders. There is a great therapeutic need for a means to specifically inhibit myostatin activity while not inhibiting or minimally inhibiting the activity of other TGF-β superfamily proteins. There is also a therapeutic need to specifically decrease the level of myostatin present in a patient while not correspondingly decreasing the level of other TGF-β superfamily proteins. In particular, a monoclonal antibody specifically reactive to myostatin (*e.g.,* specifically binds or recognizes myostatin or a portion thereof) and significantly less reactive or non-reactive with other members of the TGF-β superfamily of proteins (*e.g.*, GDF-11) may provide a particularly beneficial therapy to increase muscle mass and/or increase muscle strength. Of particular therapeutic utility are chimeric or humanized forms of such a monoclonal antibody. Myostatin is highly conserved in sequence and in function across species; therefore, not only may such an antibody be useful for the treatment of such disorders in humans, but also in other mammals including, *e.g.*, domestic animals (*e.g.*, canine and feline), sports animals (*e.g.*, equine) and food-source animals (*e.g.*, bovine, porcine, avian and ovine) particularly when framework and constant regions of the antibody substantially originate from the animal species in which the antibody is to be used therapeutically. Anti-myostatin antibodies of the invention may also be useful for treating disorders or conditions which benefit from a decrease in myostatin levels including, but not limited to, those which benefit from increasing bone density (*e.g.*, osteoporosis), Type II diabetes, metabolic syndrome, obesity, osteoarthritis, sepsis, chronic obstructive pulmonary disorder ("COPD") and disorders which are associated with muscle wasting such as renal disease, cardiac failure or disease and liver disease.

The anti-myostatin antibodies of the present invention offer advantages over other anti-myostatin antibodies in the art. The invention presents neutralizing anti-myostatin monoclonal antibodies able to bind a polypeptide consisting of amino acids at residues 40-64 *(e.g.,* SEQ ID NO: 46 for human myostatin) of the mature form of myostatin and neutralize a myostatin activity *in vitro, in vivo or in situ.* Because TGF-β family members have a high degree of homology, (*e.g.*, myostatin is about 90% homologous to GDF-11) anti-myostatin antibodies such as those of the present invention, which do not cross react or minimally cross react with GDF-11 that has an important role in establishing skeletal pattern (McPherron, A., et al., Nature Genetics, 22:260-265, 1999), are preferred for therapeutic use when compared to antibodies which cross react with GDF-11 to a greater degree.

### SUMMARY OF THE INVENTION

Anti-myostatin monoclonal antibodies, or antigen-binding fragments thereof, that specifically bind or recognize a polypeptide consisting of amino acids 40-64 of the mature form of myostatin from a mammalian source, preferably human, ANYCSGECEFVFLQKYPHTHLVHQA (SEQ ID NO: 46), or a polypeptide consisting of sequence: ANYCSGESEFVFLQKYPHTHLVHQA (SEQ ID NO: 43) are described in the present invention. Such antibodies are referred to herein as "monoclonal antibodies of the invention" or "antibodies of the invention." A monoclonal antibody of the invention may be murine, chimeric, or humanized antibodies, immunoconjugates of such antibodies, or antigen-binding fragments thereof. Preferably a monoclonal antibody of the invention exists in a homogeneous or substantially homogeneous population. Preferably, a monoclonal antibody of the invention binds myostatin (either the proprotein or the mature form of the protein, monomeric or dimeric) within the domain spanning amino acids ANYCSGECEFVFLQKYPHTHLVHQA (SEQ ID NO: 46) and thereby antagonizes or neutralizes at least one *in vitro, in vivo* or *in situ* biological activity or property associated with myostatin or a portion thereof.

According to a first aspect of the present invention there is provided an anti-myostatin monoclonal antibody or a functional fragment thereof that specifically binds a polypeptide consisting of amino acids 40-64 of a mature form of human myostatin as shown in SEQ ID NO: 46:
Ala Asn Tyr Cys Ser Gly Glu Cys Glu Phe Val Phe Leu Gln Lys Tyr
Pro His Thr His Leu Val His Gln Ala

Preferably, the monoclonal antibody or functional fragment thereof of the present invention is competitively inhibited from binding mature human myostatin by a monoclonal antibody comprising two polypeptides with sequences selected from:
(i) SEQ ID NOs: 3 and 12,
(ii) SEQ ID NOs: 4 and 13,
(iii) SEQ ID NOs: 3 and 14,
(iv) SEQ ID NOs: 5 and 12,
(v) SEQ ID NOs: 6 and 15,
(vi) SEQ ID NOs: 7 and 17,
(vii) SEQ ID NOs: 8 and 12,
(viii) SEQ ID NOs: 9 and 16,
(ix) SEQ ID NOs: 10 and 12, and
(x) SEQ ID NOs: 11 and 12.

Monoclonal antibodies of the invention preferentially bind or recognize myostatin over GDF-11, a member of the TGF-β superfamily whose mature form has about 90% amino acid homology to the mature form of myostatin. Preferably said antibodies bind myostatin with greater affinity or specificity than with which they bind GDF-11 as determined, for example, by ELISA assay, competitive ELISA assay or K_{D} values in a BIAcore^{®} assay (*e.g.*, see Example 4). Furthermore, monoclonal antibodies of the invention may have more favorable Kₒₙ, K_{off}, or Kₐ values with respect to binding myostatin than with respect to binding GDF-11. Preferably an antibody of the invention is non-cross-reactive with GDF-11 or cross-reactive at a level of 5%, 4%, 3%, 2%, 1% or less with GDF-11.

The monoclonal antibody or functional fragment thereof preferably binds myostatin with at least 20-fold greater affinity than with which it binds GDF-11.

In one embodiment, an anti-myostatin monoclonal antibody of the invention comprises a light chain variable region ("LCVR") polypeptide with an amino acid sequence selected from the group consisting of SEQ ID NO: 3, 4, 5, 6, 7, 8, 9, 10 and 11.

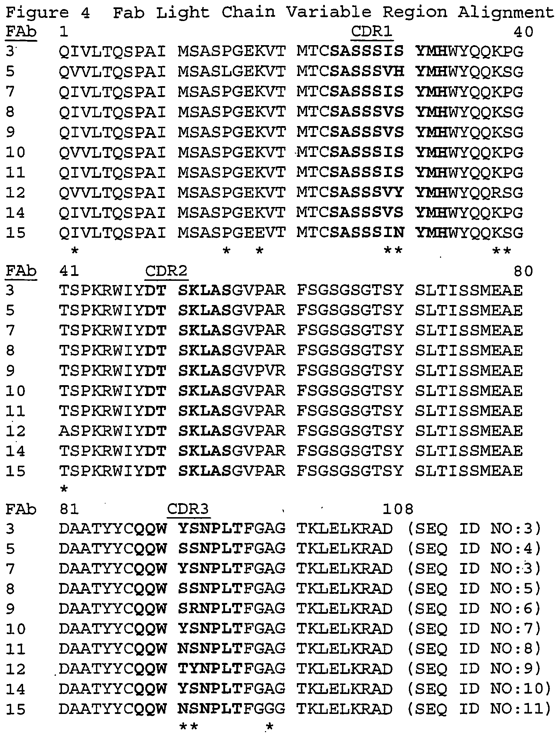

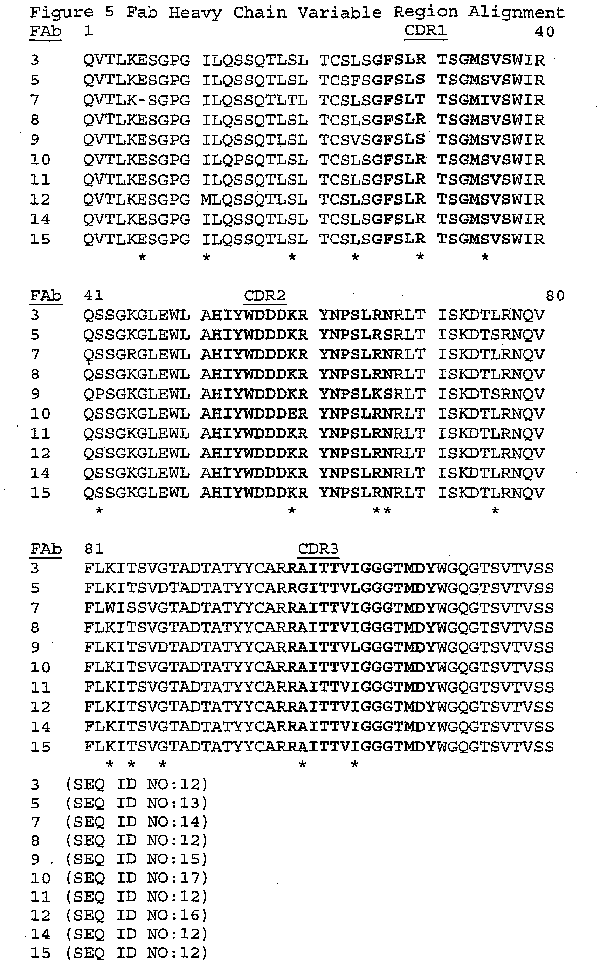

In another embodiment, an anti-myostatin monoclonal antibody of the invention comprises a heavy chain variable region ("HCVR") polypeptide with an amino acid sequence selected from the group consisting of SEQ ID NO: 12, 13, 14, 15, 16 and 17. In another embodiment, an anti-myostatin monoclonal antibody of the invention comprises a HCVR polypeptide with SEQ ID NO: 12 with amino acids 26-37 replaced with SEQ ID NO: 47, 48, 49, 50, 51, 52, 53 or 54. The sequences associated with each SEQ ID Number are shown in Tables 1 and 2 and Figures 4 and 5 herein.

In another embodiment, an anti-myostatin monoclonal antibody of the invention comprises (a) a LCVR polypeptide with an amino acid sequence selected from the group consisting of SEQ ID NO: 3, 4, 5, 6, 7, 8, 9, 10 and 11 and (b) a HCVR polypeptide with an amino acid sequence selected from the group consisting of SEQ ID NO: 12, 13, 14, 15, 16 and 17 or a HCVR polypeptide with SEQ ID NO: 12 with amino acids 26-37 replaced with SEQ ID NO: 47, 48, 49, 50, 51, 52, 53 or 54. An antibody of the invention comprising any combination of the above stated LCVR and HCVR polypeptides is contemplated, but antibodies comprising the following LCVR and HCVR combinations are preferred: (i) SEQ ID NOs: 3 and 12; (ii) SEQ ID NOs: 4 and 13; (iii) SEQ ID NOs: 3 and 14; (iv) SEQ ID NOs: 5 and 12; (v) SEQ ID NOs: 6 and 15; (vi) SEQ ID NOs: 7 and 17; (vii) SEQ ID NOs: 8 and 12; (viii) SEQ ID NOs: 9 and 16; (ix) SEQ ID NOs: 10 and 12; (x) SEQ ID NOs: 11 and 12; (xi) SEQ ID NO: 3 and SEQ ID NO: 12 with amino acids 26-37 replaced with SEQ ID NO: 47, 48, 49, 50, 51, 52, 53 or 54.

In another embodiment, a monoclonal antibody of the invention is one which can compete for binding to human myostatin or a portion of human myostatin with a competing antibody comprising two polypeptides with the sequences shown in the group consisting of: (i) SEQ ID NOs: 3 and 12, (ii) SEQ ID NOs: 4 and 13, (iii) SEQ ID NOs: 3 and 14, (iv) SEQ ID NOs: 5 and 12, (v) SEQ ID NOs: 6 and 15, (vi) SEQ ID NOs: 7 and 17, (vii) SEQ ID NOs: 8 and 12, (viii) SEQ ID NOs: 9 and 16, (ix) SEQ ID NOs: 10 and 12, (x) SEQ ID NOs: 11 and 12, and (xi) SEQ ID NO: 3 and SEQ ID NO: 12 with amino acids 26-37 replaced with SEQ ID NO: 47, 48, 49, 50, 51, 52, 53 or 54.

In another embodiment, a LCVR of an anti-myostatin monoclonal antibody of the invention comprises 1, 2 or 3 peptides selected from the group consisting of peptides with a sequence as shown in SEQ ID NOs: 38, 23 and 56 (see Table 1). Preferably a peptide with the sequence shown in SEQ ID NO: 38, when present in said antibody, is at LCVR CDR1. Preferably, a peptide with the sequence shown in SEQ ID NO: 23, when present in said antibody, is at LCVR CDR2. Preferably, a peptide with the sequence shown in SEQ ID NO: 56, when present in said antibody, is at LCVR CDR3.

In another embodiment, a LCVR of an anti-myostatin monoclonal antibody of the invention comprises 1, 2 or 3 peptides selected from the group consisting of peptides with a sequence as shown in (a) SEQ ID NO: 18, 19, 20, 21 or 22; (b) SEQ ID NO: 23, and (c) SEQ ID NO: 24, 25, 26, 27 or 28. Preferably, a peptide with the sequence shown in SEQ ID NO: 18, 19, 20, 21, or 22, when present in an antibody of the invention, is at LCVR CDR1. Preferably a peptide with the sequence shown in SEQ ID NO: 23, when present in an antibody of the invention, is at LCVR CDR2. Preferably a peptide with the sequence shown in SEQ ID NO: 24, 25, 26, 27 or 28, when present in an antibody of the invention, is at LCVR CDR3. The LCVR will further comprise framework sequence. In a humanized antibody for therapeutic use in humans, the framework sequence may be substantially of human origin. In an antibody for use in a non-human animal, the framework region sequence may substantially originate from the genome of the animal in which it is to be used therapeutically

In another embodiment, a HCVR of an anti-myostatin monoclonal antibody of the invention comprises 1, 2 or 3 peptides selected from the group consisting of peptides with a sequence as shown in SEQ ID NOS: 55, 41 and 42 (see Table 2). Preferably a peptide with the sequence shown in SEQ ID NO: 55, when present in said antibody, is at HCVR CDR1. Preferably, a peptide with the sequence shown in SEQ ID NO: 41, when present in said antibody, is at HCVR CDR2. Preferably, a peptide with the sequence shown in SEQ ID NO: 42, when present in said antibody, is at HCVR CDR3.

In another embodiment, a HCVR of an anti-myostatin monoclonal antibody of the invention comprises 1, 2 or 3 peptides selected from the group consisting of peptides with a sequence as shown in (a) SEQ ID NO: 29, 30, 31, 47, 48, 49, 50, 51, 52, 53 or 54; (b) SEQ ID NO: 32, 33, 34, or 35; and (c) 36 or 37. Preferably a peptide with the sequence shown in SEQ ID NO: 29, 30, 31, 47, 48, 49, 50, 51, 52, 53 or 54, when present in an antibody of the invention, is at HCVR CDR1. Preferably a peptide with the sequence shown in SEQ ID NO: 32, 33, 34, or 35, when present in an antibody of the invention, is at HCVR CDR2. Preferably a peptide with the sequence shown in SEQ ID NO: 36 or 37, when present in an antibody of the invention, is at HCVR CDR3. The HCVR will further comprise framework sequence. In a humanized antibody for therapeutic use in humans, the framework sequence may be substantially of human origin. In an antibody for use in a non-human animal, the framework sequence may substantially originate from the genome of the animal in which it is to be used therapeutically.

One embodiment of the invention provides an anti-myostatin monoclonal antibody comprising the six peptides with the sequences shown in SEQ ID NOs: 38, 23, 56, 55, 41 and 42. Preferably, in said antibody, the peptide with the sequence shown in SEQ ID NO: 38 is located at LCVR CDR1, the peptide with the sequence shown in SEQ ID NO: 23 is located at LCVR CDR2, the peptide with the sequence shown in SEQ ID NO: 56 is located at LCVR CDR3, the peptide with the sequence shown in SEQ ID NO: 55 is located at HCVR CDR1, the peptide with the sequence shown in SEQ ID NO: 41 is located at HCVR CDR2, and the peptide with the sequence shown in SEQ ID NO: 42 is located at HCVR CDR3.

Another embodiment provides an anti-myostatin monoclonal antibody comprising the six peptides with the sequences as shown in SEQ ID NOs: (i) 18; (ii) 23; (iii) 24; (iv) 29, 47, 48, 49, 50, 51, 52, 53 or 54; (v) 32; and (vi) 36 Preferably, in said antibody, the peptide with the sequence shown in SEQ ID NO: 18 is located at LCVR CDR1, the peptide with the sequence shown in SEQ ID NO: 23 is located at LCVR CDR2, the peptide with the sequence shown in SEQ ID NO: 24 is located at LCVR CDR3, the peptide with the sequence shown in SEQ ID NO: 29, 47, 48, 49, 50, 51, 52, 53 or 54 is located at HCVR CDR1, the peptide with the sequence shown in SEQ ID NO: 32 is located at HCVR CDR2, and the peptide with the sequence shown in SEQ ID NO: 36 is located at HCVR CDR3.

Another embodiment provides an anti-myostatin monoclonal antibody comprising the six peptides with the sequences as shown in SEQ ID NOs: 19, 23, 25, 30, 33 and 37 Preferably, in said antibody, the peptide with the sequence shown in SEQ ID NO: 19 is located at LCVR CDR1, the peptide with the sequence shown in SEQ ID NO: 23 is located at LCVR CDR2, the peptide with the sequence shown in SEQ ID NO: 25 is located at LCVR CDR3, the peptide with the sequence shown in SEQ ID NO: 30 is located at HCVR CDR1, the peptide with the sequence shown in SEQ ID NO: 33 is located at HCVR CDR2, and the peptide with the sequence shown in SEQ ID NO: 37 is located at HCVR CDR3.

Another embodiment provides an anti-myostatin monoclonal antibody comprising the six peptides with the sequences as shown in SEQ ID NOs: 18, 23, 24, 31, 32 and 36 Preferably, in said antibody, the peptide with the sequence shown in SEQ ID NO: 18 is located at LCVR CDR1, the peptide with the sequence shown in SEQ ID NO: 23 is located at LCVR CDR2, the peptide with the sequence shown in SEQ ID NO: 24 is located at LCVR CDR3, the peptide with the sequence shown in SEQ ID NO: 31 is located at HCVR CDR1, the peptide with the sequence shown in SEQ ID NO: 32 is located at HCVR CDR2, and the peptide with the sequence shown in SEQ ID NO: 36 is located at HCVR CDR3.

Another embodiment provides an anti-myostatin monoclonal antibody comprising the six peptides with the sequences as shown in SEQ ID NOs: 20, 23, 25, 29, 32 and 36. Preferably, in said antibody, the peptide with the sequence shown in SEQ ID NO: 20 is located at LCVR CDR1, the peptide with the sequence shown in SEQ ID NO: 23 is located at LCVR CDR2, the peptide with the sequence shown in SEQ ID NO: 25 is located at LCVR CDR3, the peptide with the sequence shown in SEQ ID NO: 29 is located at HCVR CDR1, the peptide with the sequence shown in SEQ ID NO: 32 is located at HCVR CDR2, and the peptide with the sequence shown in SEQ ID NO: 36 is located at HCVR CDR3.

Another embodiment provides an anti-myostatin monoclonal antibody comprising the six peptides with the sequences as shown in SEQ ID NOs: 20, 23, 26, 30, 34 and 36. Preferably, in said antibody, the peptide with the sequence shown in SEQ ID NO: 20 is located at LCVR CDR1, the peptide with the sequence shown in SEQ ID NO: 23 is located at LCVR CDR2, the peptide with the sequence shown in SEQ ID NO: 26 is located at LCVR CDR3, the peptide with the sequence shown in SEQ ID NO: 30 is located at HCVR CDR1, the peptide with the sequence shown in SEQ ID NO: 34 is located at HCVR CDR2, and the peptide with the sequence shown in SEQ ID NO: 36 is located at HCVR CDR3.

Another embodiment provides an anti-myostatin monoclonal antibody comprising the six peptides with the sequences as shown in SEQ ID NOs: 18, 23, 24, 29, 35 and 36. Preferably, in said antibody, the peptide with the sequence shown in SEQ ID NO: 18 is located at LCVR CDR1, the peptide with the sequence shown in SEQ ID NO: 23 is located at LCVR CDR2, the peptide with the sequence shown in SEQ ID NO: 24 is located at LCVR CDR3, the peptide with the sequence shown in SEQ ID NO: 29 is located at HCVR CDR1, the peptide with the sequence shown in SEQ ID NO: 35 is located at HCVR CDR2, and the peptide with the sequence shown in SEQ ID NO: 36 is located at HCVR CDR3.

Another embodiment provides an anti-myostatin monoclonal antibody comprising the six peptides with the sequences as shown in SEQ ID NOs: 18, 23, 27, 29, 32 and 36. Preferably, in said antibody, the peptide with the sequence shown in SEQ ID NO: 18 is located at LCVR CDR1, the peptide with the sequence shown in SEQ ID NO: 23 is located at LCVR CDR2, the peptide with the sequence shown in SEQ ID NO: 27 is located at LCVR CDR3, the peptide with the sequence shown in SEQ ID NO: 29 is located at HCVR CDR1, the peptide with the sequence shown in SEQ ID NO: 32 is located at HCVR CDR2, and the peptide with the sequence shown in SEQ ID NO: 36 is located at HCVR CDR3.

Another embodiment provides an anti-myostatin monoclonal antibody comprising the six peptides with the sequences as shown in SEQ ID NOs: 21, 23, 28, 29, 32, 36 Preferably, in said antibody, the peptide with the sequence shown in SEQ ID NO: 21 is located at LCVR CDR1, the peptide with the sequence shown in SEQ ID NO: 23 is located at LCVR CDR2, the peptide with the sequence shown in SEQ ID NO: 28 is located at LCVR CDR3, the peptide with the sequence shown in SEQ ID NO: 29 is located at HCVR CDR1, the peptide with SEQ ID NO: 32 is located at HCVR CDR2, and the peptide with the sequence shown in SEQ ID NO: 36 is located at HCVR CDR3.

Another embodiment provides an anti-myostatin monoclonal antibody comprising the six peptides with the sequences as shown in SEQ ID NOs: 20, 23, 24, 29, 32 and 36. Preferably, in said antibody, the peptide with the sequence shown in SEQ ID NO: 20 is located at LCVR CDR1, the peptide with the sequence shown in SEQ ID NO: 23 is located at LCVR CDR2, the peptide with the sequence shown in SEQ ID NO: 24 is located at LCVR CDR3, the peptide with the sequence shown in SEQ ID NO: 29 is located at HCVR CDR1, the peptide with the sequence shown in SEQ ID NO: 32 is located at HCVR CDR2, and the peptide with the sequence shown in SEQ ID NO: 36 is located at HCVR CDR3.

Another embodiment provides an anti-myostatin monoclonal antibody comprising the six peptides with the sequences as shown in SEQ ID NOs: 22, 23, 27, 29, 32 and 36. Preferably, in said antibody, the peptide with the sequence shown in SEQ ID NO: 22 is located at LCVR CDR1, the peptide with the sequence shown in SEQ ID NO: 23 is located at LCVR CDR2, the peptide with the sequence shown in SEQ ID NO: 27 is located at LCVR CDR3, the peptide with the sequence shown in SEQ ID NO: 29 is located at HCVR CDR1, the peptide with the sequence shown in SEQ ID NO: 32 is located at HCVR CDR2, and the peptide with the sequence shown in SEQ ID NO: 36 is located at HCVR CDR3.

An anti-myostatin monoclonal antibody of the invention may further comprise a heavy chain constant region selected from the group consisting of IgG₁, IgG₂, IgG₃, IgG₄, IgA, IgE, IgM and IgD. An anti-myostatin monoclonal antibody of the invention may further comprise a kappa or lambda light chain constant region. When the antibody is to be used as a human therapeutic, the constant region is preferably substantially of human origin. When the antibody is to be used as a therapeutic in a non-human animal, the constant region preferably substantially originates from the animal in which the antibody is to be used as a therapeutic.

An anti-myostatin monoclonal antibody of the invention may comprise or consist of an intact antibody (*i.e.*, full length), a substantially intact antibody, a Fab fragment, a F(ab')₂ fragment or a single chain Fv fragment.

In a preferred embodiment, an anti-myostatin monoclonal antibody of the invention is a chimeric antibody. In a more preferred embodiment, an anti-myostatin monoclonal antibody of the invention is a humanized antibody in which framework sequence and constant region sequence present in the antibody is substantially of human origin. The humanized antibody is preferably a full-length antibody. Alternatively, the framework region, or a portion thereof, and any constant region present in the antibody may substantially originate from the genome of the animal in which the antibody is to be used as a therapeutic, e.g., domestic animals (*e.g.*, canine, feline), sports animals (*e.g.*, equine) and food-source animals (*e.g.*, bovine, porcine, avian and ovine).

In another embodiment, the invention provides an isolated nucleic acid molecule that comprises a nucleic acid that encodes an LCVR of an antibody of the invention, an HCVR of an antibody of the invention or an anti-myostatin monoclonal antibody of the invention. An exemplary polynucleotide encoding an LCVR of the invention has the sequence shown in SEQ ID NO: 44. An exemplary polynucleotide encoding an HCVR of the invention has the sequence shown in SEQ ID NO: 45.

In another embodiment, the invention provides a vector, preferably (but not limited to) a plasmid, a recombinant expression vector, a yeast expression vector, or a retroviral expression vector comprising a polynucleotide encoding an anti-myostatin monoclonal antibody of the invention. Alternatively, a vector of the invention comprises a polynucleotide encoding an LCVR and/or a polynucleotide encoding an HCVR of the invention. When both an LCVR and an HCVR encoding sequence are present in the same vector, they may be transcribed from one promoter to which they are both operably linked or they may be transcribed independently, each from a separate promoter to which it is operable linked. If the sequences encoding LCVR and HCVR are present in the same vector and transcribed from one promoter to which they are both operably linked, the LCVR may be 5' to the HCVR or the LCVR may be 3' to the HCVR, furthermore the LCVR and HCVR coding region in the vector may be separated by a linker sequence of any size or content, preferably such linker, when present, is a polynucleotide encoding an internal ribosome entry site.

In another embodiment, the invention provides a host cell comprising a nucleic acid molecule of the present invention. Preferably a host cell of the invention comprises one or more vectors or constructs comprising a nucleic acid molecule of the present invention. The host cell of the invention is a cell into which a vector of the invention has been introduced (*e.g.*, via transformation, transduction, infection), said vector comprising a polynucleotide encoding a LCVR of an antibody of the invention and/or a polynucleotide encoding a HCVR of the invention. The invention also provides a host cell into which two vectors of the invention have been introduced; one comprising a polynucleotide encoding a LCVR of an antibody of the invention and one comprising a polynucleotide encoding a HCVR present in an antibody of the invention and each operably linked to a promoter sequence. The host cell types include mammalian, bacterial, plant and yeast cells. Preferably the host cell is a CHO cell, a COS cell, a SP2/0 cell, a NS0 cell, a yeast cell or a derivative or progeny of any preferred cell type.

In another embodiment, the invention provides a method of preparing an anti-myostatin monoclonal antibody of the invention comprising maintaining a host cell of the invention (*i***.***e*., host cell that has been transformed, transduced or infected with a vector (or vectors) of the invention) under conditions appropriate for expression of a monoclonal antibody of the invention, whereby such antibody is expressed. The method may further comprise the step of isolating the monoclonal antibody of the invention from the cell or preferably from the culture media in which said cell is grown.

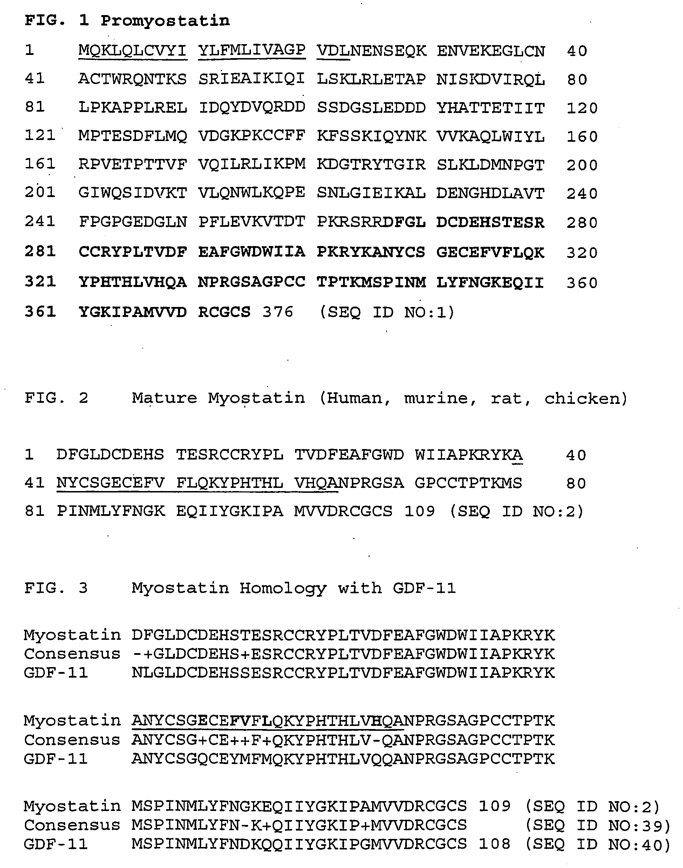

The invention embodies the process of producing an antibody of the invention by injecting a non-human animal, preferably a rodent, more preferably a mouse, with (i) an immunogenic peptide consisting of a peptide with a sequence as shown in SEQ ID NOs: 46 or 43, or (ii) an immunogenic peptide consisting of 24, 23, 22, 21, 20, 19, 18, 17, 16, 15, 14, 13, 12, 11, 10, 9, 8, 7, 6 or 5 contiguous amino acids of a peptide with a sequence as shown in SEQ ID NOs: 46 or 43, preferably said immunogenic peptide spans amino acid residues in which 1, 2, 3, 4 or 5 of said contiguous amino acids are selected from the group consisting of amino acids at residue numbers 46, 49, 50, 52 and 62 of mature myostatin where the amino acid at said residue number differs from the amino acid present at the equivalent position of GDF-11 (See, Fig. 3), or (iii) an immunogenic peptide consisting of amino acids at postions 40-64 of the mature form of myostatin of any mammal, or (iv) an immunogenic peptide consisting of 24, 23, 22, 21, 20, 19, 18, 17, 16, 15, 14, 13, 12, 11, 10, 9, 8, 7, 6 or 5 contiguous amino acids of a peptide consisting of the amino acids at positions 40-64 of the mature form of myostatin of any mammal, preferably said immunogenic peptide spans amino acid residues in which 1, 2, 3, 4 or 5 of said contiguous amino acids are amino acids which differ from the amino acid present at the equivalent position of GDF-11 in the same mammal. Anti-myostatin monoclonal antibodies are generated from the immunized animals using any method known in the art, preferably by hybridoma synthesis. The anti-myostatin monoclonal antibodies are screened by any method available in the art (*e.g.*, phage display, ribosome display, yeast display, bacterial display, ELISA assay) for binding to mature myostatin, or a portion thereof comprising the immunogenic peptide, or to the immunogenic peptide. Optionally, the anti-myostatin monoclonal antibodies are screened by any method available in the art for binding to mature GDF-11 or a portion thereof. Anti-myostatin monoclonal antibodies are selected which specifically or preferentially bind myostatin with respect to GDF-11. The invention further embodies a monoclonal antibody made by this process. Preferably said monoclonal antibody binds myostatin at least 5, 10, 20, 30, 40, 50, 60, 70, 80, 90, or 100-fold greater than with which it binds GDF-11; more preferably at least 150, 200, 250, 300, 350, 400, 450, 500, 550 or 600-fold greater than with which it binds GDF-11, as determined by a method know to one of skill in the art e.g., by ELISA, competition ELISA or K_{D} values in a BIAcore^{®} assay. Most preferably the monoclonal antibodies do not bind GDF-11 above background levels in any binding assay available in the art.

The invention also embodies the process of producing an antibody of the invention by injecting a non-human animal, preferably a rodent, more preferably a mouse, with (i) an immunogenic peptide comprising a sequence as shown in SEQ ID NOs: 46 or 43, or (ii) an immunogenic peptide comprising 24, 23, 22, 21, 20, 19, 18, 17, 16, 15, 14, 13, 12, 11, 10, 9, 8, 7, 6 or 5 contiguous amino acids of a peptide consisting of a sequence as shown in SEQ ID NOs: 46 or 43, preferably said immunogenic peptide spans amino acid residues in which 1, 2, 3, 4 or 5 of said contiguous amino acids are selected from the group consisting of amino acids at residue numbers 46, 49, 50, 52 and 62 of mature myostatin where the amino acid at said residue number differs from the amino acid present at the equivalent position of GDF-11 (See, Fig. 3), or (iii) an immunogenic peptide comprising amino acids at positions 40-64 of the mature form of myostatin of any mammal, or (iv) an immunogenic peptide comprising 24, 23, 22, 21, 20, 19, 18, 17, 16, 15, 14, 13, 12, 11, 10, 9, 8, 7, 6 or 5 contiguous amino acids of a peptide consisting of the amino acids at positions 40-64 of the mature form of myostatin of any mammal, preferably said immunogenic peptide spans amino acid residues in which 1, 2, 3, 4 or 5 of said contiguous amino acids are amino acids which differ from the amino acid present at the equivalent position of GDF-11 in the same mammal. Anti-myostatin monoclonal antibodies are generated from the immunized animals using any method known in the art, preferably by hybridoma synthesis. The ant-myostatin monoclonal antibodies are screened by any method available in the art (e.g., phage display, ribosome display, yeast display, bacterial display, ELISA assay) for binding to (i) an antigenic peptide consisting of a sequence as shown in SEQ ID NOs: 46 or 43, or (ii) an antigenic peptide consisting of 24, 23, 22, 21, 20, 19, 18, 17, 16, 15, 14, 13, 12, 11, 10, 9, 8, 7, 6 or 5 contiguous amino acids of a peptide consisting of a sequence as shown in SEQ ID NOs: 46 or 43, preferably said peptide spans amino acid residues in which 1, 2, 3, 4 or 5 of said contiguous amino acids are selected from the group consisting of amino acids at residue numbers 46, 49, 50, 52 and 62 of mature myostatin where the amino acid at said residue number differs from the amino acid present at the equivalent position of GDF-11 (See, Fig. 3), or (iii) an antigenic peptide consisting of the amino acids at positions 40-64 of the mature form of myostatin of any mammal, or (iv) an antigenic peptide consisting of 24, 23, 22, 21, 20, 19, 18, 17, 16, 15, 14, 13, 12, 11, 10, 9, 8, 7, 6 or 5 contiguous amino acids of a peptide consisting of the amino acids at positions 40-64 of the mature form of myostatin of any mammal, preferably said immunogenic peptide spans amino acid residues in which 1, 2, 3, 4 or 5 of said contiguous amino acids are amino acids which differ from the amino acid present at the equivalent position of GDF-11 in the same mammal. Optionally, the anti-myostatin monoclonal antibodies are screened by any method available in the art for binding to mature GDF-11 or a portion thereof. Anti-myostatin monoclonal antibodies are selected which specifically or preferentially bind myostatin with respect to GDF-11. The invention further embodies a monoclonal antibody made by this process. Preferably said monoclonal antibody binds myostatin at least 5, 10, 20, 30, 40, 50, 60, 70, 80, 90, or 100-fold greater than with which it binds GDF-11; more preferably at least 150, 200, 250, 300, 350, 400, 450, 500, 550 or 600-fold greater than with which it binds GDF-11, as determined by a method know to one of skill in the art e.g., by ELISA, competition ELISA or K_{D} values in a BIAcore^{®} assay. Most preferably the monoclonal antibodies do not bind GDF-11 above background levels in any binding assay available in the art.

It is contemplated that said antibody made by any process of the invention may be further altered into a chimeric antibody in which at least a portion of the framework and/or constant region originates from a mammal different from that which was immunized to generate the monoclonal antibody and still fall within the scope of the invention. The antibodies of the invention may be humanized in which the murine CDR regions exist within a substantially human framework region, and the constant region, to the extent it is present in the antibody, is also substantially of human origin. The antibodies of the invention may be such that the murine CDR regions exist within a framework region and constant region (to the extent it is present in the antibody) originates from the germline sequence of the animal in which the antibody is to be used therapeutically.

Various forms of the antibodies of the invention are contemplated herein. For example, an anti-myostatin monoclonal antibody of the invention may be a full-length antibody (*e.g.*, having an immunoglobulin constant region) or an antibody fragment (e.g., a F(ab')2). It is understood that all such forms of the antibodies are encompassed herein within the term "antibody." Furthermore, the antibody may be labeled with a detectable label, immobilized on a solid phase and/or conjugated with a heterologous compound (*e.g*., an enzyme or toxin) according to methods known in the art.

Diagnostic uses for monoclonal antibodies of the invention are contemplated. In one diagnostic application, the invention provides a method for determining the presence of myostatin protein comprising exposing a test sample suspected of containing the myostatin protein to an anti-myostatin antibody of the invention and determining specific binding of the antibody to the sample. An anti-myostatin antibody of the invention may be used to determine the levels of myostatin in test samples by comparing test sample values to a stand curve generated by binding said antibody to samples with known amounts of myostatin. The invention further provides a kit comprising an antibody of the invention and, preferably, instructions for using the antibody to detect myostatin protein in *e.g.,* a test sample.

In another embodiment, the invention provides a pharmaceutical composition comprising an anti-myostatin monoclonal antibody of the invention. The pharmaceutical composition of the invention may further comprise a pharmaceutically acceptable carrier. In said pharmaceutical composition, the anti-myostatin monoclonal antibody of the invention is the active ingredient. Preferably the pharmaceutical composition comprises a homogeneous or substantially homogeneous population of an anti-myostatin monoclonal antibody of the invention. The composition for therapeutic use is sterile and may be lyophilized.

The invention provides a method of inhibiting myostatin activity in a mammal, preferably a human, in need thereof comprising administering a therapeutically effective amount, or prophylactically effective amount, of an anti-myostatin monoclonal antibody of the invention to said mammal. The invention further provides a method of treating or preventing a disease or disorder ameliorated by the inhibition of signal transduction resulting from the binding of myostatin to its receptor that comprises administering to a patient (*e.g.*, a human) in need of such treatment or prevention a therapeutically or prophylactically effective amount of a monoclonal antibody of the invention. As used herein, "treating or preventing" refers to a disease or disorder associated with abnormal myostatin levels or benefited by inhibiting a myostatin activity or benefited by a change in the existing myostatin level. Diseases or disorders treated or prevented with an antibody of the invention include, but are not limited to, frailty, cachexia, age-related sarcopenia, muscle wasting, myopathy, muscular dystrophy, osteoporosis, obesity, COPD, renal failure or disease, liver failure or disease, cardiac failure or disease, metabolic syndrome and Type II diabetes. The invention further provides a method for increasing muscle mass, increasing muscle strength, and increasing bone density in a mammal, preferably a human, in need thereof by administering a therapeutically effective amount of an anti-myostatin monoclonal antibody of the invention.

According to another aspect of the present invention there is provided a monoclonal antibody or a functional fragment thereof for use as a medicament.

Preferably, the monoclonal antibody or a functional fragment thereof of the present invention is for use in the prevention or treatment of muscle wasting, myopathy, muscular dystrophy, muscle weakness, frailty, cachexia, renal failure or disease, liver failure or disease, cardiac failure or disease, type II diabetes, metabolic syndrome, osteoporosis, obesity or COPD in a human. More preferably, the monoclonal antibody or a functional fragment thereof of the present invention is for use in increasing muscle mass in a mammal.

The invention embodies an anti-myostatin monoclonal antibody of the invention for use in the manufacture of a medicament for administration to a mammal, preferably a human, for the treatment of e.g., frailty, cachexia, age-related sarcopenia, muscle wasting, myopathy, muscular dystrophy, osteoporosis, obesity, COPD, renal failure or disease, liver failure or disease, cardiac failure or disease, metabolic syndrome and Type II diabetes in a mammal, preferably a human, in need thereof by administering to said mammal a therapeutically effective or prophylactically effective amount of an anti-myostatin monoclonal antibody of the invention.

The invention embodies an article of manufacture comprising a packaging material and an antibody of the invention contained within said packaging material and wherein the packaging material comprises a package insert which indicates that the antibody specifically neutralizes a myostatin activity or decreases the level of myostatin. Optionally, the package insert further indicates that the antibody preferentially neutralizes a myostatin activity with respect to a GDF-11 activity or preferentially decreases the level of myostatin with respect to decreasing the level of GDF-11 by preferentially binding myostatin with respect to binding GDF-11.

**Table 1 CDR Sequences - Light Chain Variable Region (LCVR)**

| FAb | CDR1 | CDR2 | CDR3 |
|---|---|---|---|
| 3 | SASSSISYMH (SEQ ID NO: 18) | DTSKLAS (SEQ ID NO: 23) | QQWYSNPLT (SEQ ID NO: 24) |
| 5 | SASSSVHYMH (SEQ ID NO: 19) | DTSKLAS (SEQ ID NO: 23) | QQWSSNPLT (SEQ ID NO: 25) |
| 7 | SASSSISYMH (SEQ ID NO: 18) | DTSKLAS (SEQ ID NO: 23) | QQWYSNPLT (SEQ ID NO: 24) |
| 8 | SASSSVSYMH (SEQ ID NO: 20) | DTSKLAS (SEQ ID NO: 23) | QQWSSNPLT (SEQ ID NO: 25) |
| 9 | SASSSVSYMH (SEQ ID NO: 20) | DTSKLAS (SEQ ID NO: 23) | QQWSRNPLT (SEQ ID NO: 26) |
| 10 | SASSSISYMH (SEQ ID NO: 18) | DTSKLAS (SEQ ID NO: 23) | QQWYSNPLT (SEQ ID NO: 24) |
| 11 | SASSSISYMH (SEQ ID NO: 18) | DTSKLAS (SEQ ID NO: 23) | QQWNSNPLT (SEQ ID NO: 27) |
| 12 | SASSSVYYMH (SEQ ID NO: 21) | DTSKLAS (SEQ ID NO: 23) | QQWTYNPLT (SEQ ID NO: 28) |
| 14 | SASSSVSYMH (SEQ ID NO: 20) | DTSKLAS (SEQ ID NO: 23) | QQWYSNPLT (SEQ ID NO: 24) |
| 15 | SASSSINYMH (SEQ ID NO: 22) | DTSKLAS (SEQ ID NO: 23) | QQWNSNPLT (SEQ ID NO: 27) |
| Consensus | SASSSX₂₉X₃₀*YMH (SEQ ID NO: 38) | DTSKLAS (SEQ ID NO: 23) | QQWX₉₁X₉₂NPLT** (SEQ ID NO: 56) |

| | | | |
|---|---|---|---|
| * X₂₉ is a hydrophobic amino acid, X₃₀ is S, T, H, Y or N **X₉₁ is Y, S, N or T, X₉₂ is R, K, Y, S or T | | | |

**Table 2 CDR Sequence - Heavy Chain Variable Region (HCVR)**

| FAb | CDR1 | CDR2 | CDR3 |
|---|---|---|---|
| 3 | GFSLRTSGMSVS (SEQ ID NO: 29) | HIYWDDDKRYNPSLRN (SEQ ID NO: 32) | RAITTVIGGGTMDY (SEQ ID NO: 36) |
| 5 | GFSLSTSGMSVS (SEQ ID NO: 30) | HIYWDDDKRYNPSLRS (SEQ ID NO: 33) | RGITTVLGGGTMDY (SEQ ID NO: 37) |
| 7 | GFSLTTSGMIVS (SEQ ID NO: 31) | HIYWDDDKRYNPSLRN (SEQ ID NO: 32) | RAITTVIGGGTMDY (SEQ ID NO: 36) |
| 8 | GFSLRTSGMSVS (SEQ ID NO: 29) | HIYWDDDKRYNPSLRN (SEQ ID NO: 32) | RAITTVIGGGTMDY (SEQ ID NO: 36) |
| 9 | GFSLSTSGMSVS (SEQ ID NO: 30) | HIYWDDDKRYNPSLKS (SEQ ID NO: 34) | RAITTVIGGGTMDY (SEQ ID NO: 36) |
| 10 | GFSLRTSGMSVS (SEQ ID NO: 29) | HIYWDDDERYNPSLRN (SEQ ID NO: 35) | RAITTVIGGGTMDY (SEQ ID NO: 36) |
| 11 | GFSLRTSGMSVS (SEQ ID NO: 29) | HIYWDDDKRYNPSLRN (SEQ ID NO: 32) | RAITTVIGGGTMDY (SEQ ID NO: 36) |
| 12 | GFSLRTSGMSVS (SEQ ID NO: 29) | HIYWDDDKRYNPSLRN (SEQ ID NO: 32) | RAITTVIGGGTMDY (SEQ ID NO: 36) |
| 14 | GFSLRTSGMSVS (SEQ ID NO: 29) | HIYWDDDKRYNPSLRN (SEQ ID NO: 32) | RAITTVIGGGTMDY (SEQ ID NO: 36) |
| 15 | GFSLRTSGMSVS (SEQ ID NO: 29) | HIYWDDDKRYNPSLRN (SEQ ID NO: 32) | RAITTVIGGGTMDY (SEQ ID NO: 36) |
| 16 | GFSLRTSGSSVS (SEQ ID NO: 47) | HIYWDDDKRYNPSLRN (SEQ ID NO: 32) | RATTTVIGGGTMDY (SEQ ID NO: 36) |
| 17 | GFSLRKSGMSVS (SEQ ID NO: 48) | HIYWDDDKRYNPSLRN (SEQ ID NO: 32) | RAITTVIGGGTMDY (SEQ ID NO: 36) |
| 18 | GFSLRTVGMSVS (SEQ ID NO: 49) | HIYWDDDKRYNPSLRN (SEQ ID NO: 32) | RAITTVIGGGTMDY (SEQ ID NO: 36) |
| 19 | GFSLRTLGMSVS (SEQ ID NO: 50) | HIYWDDDKRYNPSLRN (SEQ ID NO: 32) | RAITTVIGGGTMDY (SEQ ID NO: 36) |
| 20 | GFSLRTLGSSVS (SEQ ID NO: 51) | HIYWDDDKRYNPSLRN (SEQ ID NO: 32) | RAITTVIGGGTMDY (SEQ ID NO: 36) |
| 21 | GFSLRKVGSSVS (SEQ ID NO: 52) | HIYWDDDKRYNPSLRN (SEQ ID NO: 32) | RAITTVIGGGTMDY (SEQ ID NO: 36) |
| 22 | GFSLRKLGSSVS (SEQ ID NO: 53) | HIYWDDDKRYNPSLRN (SEQ ID NO: 32) | RAITTVIGGGTMDY (SEQ ID NO: 36) |
| 23 | GFSLRKSGSSVS (SEQ ID NO: 54) | HIYWDDDKRYNPSLRN (SEQ ID NO: 32) | RAITTVIGGGTMDY (SEQ ID NO: 36) |
| Consensus | GFSLX₅X₆X₇GX₉X₁₀VS * (SEQ ID NO: 55) | HIYWDDDX₈RYNPSLX₁₅ X₁₆** (SEQ ID NO: 41) | RX₂ITTVX₇GGGTM DY*** (SEQ ID NO: 42) |

| | | | |
|---|---|---|---|
| *X₅ is R, K,T or S; X 6 is T or K, X₇ is S, V or L, X₉ is M or S, X₁₀ is S, T, I, L or V **X₈ is K, R, E or D; X₁₅ is K or R; X₁₆ is S, T, N or Q ***X₂ is A or G; X₇ is I, L or V | | | |

### BRIEF DESRIPTION OF THE DRAWINGS

FIG. 1 shows the amino acid sequence of human promyostatin with the signal sequence underlined and the portion of the protein at the carboxy-terminus that makes up a monomer of the mature form of myostatin in bold letters.
FIG. 2 shows the amino acid sequence of human mature myostatin. The antigenic epitope of the present invention is underlined.
FIG. 3 shows the alignment of the amino acid sequence of the mature form human myostatin and human GDF-11 with the antigenic epitope of the present invention underlined, the residues within the antigenic epitope that differ between myostatin and GDF-11 in bold print. The symbol (+) indicates a conservative amino acid difference between myostatin and GDF-11 at that position while the symbol (-) indicates a non-conservative amino acid difference between myostatin and GDF-11 at that position.
FIG. 4 shows the alignment of the LCVR of Fabs 3, 5, 7, 8, 9, 10, 11, 12, 14 and 15 with the CDR domains in bold print. The symbol (*) indicates an amino acid residue where there is variance among the Fabs.
FIG. 5 shows the alignment of the HCVR of Fabs 3, 5, 7, 8, 9, 10, 11, 12, 14 and 15 with the CDR domains in bold print. The symbol (*) indicates an amino acid residue where there is variance among the Fabs.

### DETAILED DESCRIPTION OF THE INVENTION

The present invention relates to monoclonal antibodies or functional fragments thereof (e.g., an antigen-binding fragment) which specifically bind to a mammalian myostatin or portion thereof. The antigenic epitope to which monoclonal antibodies of the invention bind is localized to residues 40-64 of mature myostatin. In one embodiment, a monoclonal antibody of the invention blocks binding of a ligand (e.g., myostatin receptor) to myostatin or inhibits a biological activity of myostatin.

The antibodies of the invention specifically bind mature myostatin or a portion thereof with an affinity of at least about 1 x 10⁻⁷ M, preferably at least about 9 x 10⁻⁸ M or 7 x 10⁻⁸ M, and more preferably at least about 5 x 10⁻⁸ M. Preferably the antibodies of the invention do not bind GDF-11 greater than background levels of any standard binding assay known in the art. In one embodiment, antibodies of the invention demonstrate inhibition of a myostatin biological activity *in retro* or *in vivo* at less than 150 µg/ml, preferably less than 100 µg/ml, more preferably less than 90, 80, 70, 60 or 50 µg/ml, and even more preferably less than about 20 µg/ml, and even more preferably less than about 2 or 0.2 or 0.02 µg/ml. When used herein, the term "mature myostatin" may refer to the monomeric or the dimeric form, preferably homodimeric, of the protein resulting after proteolytic cleavage of the proprotein form of myostatin.

A full-length antibody as it exists naturally is an immunoglobulin molecule comprised of four peptide chains, two heavy (H) chains (about 50-70 kDa when full length) and two light (L) chains (about 25 kDa when full length) interconnected by disulfide bonds. The amino terminal portion of each chain includes a variable region of about 100-110 or more amino acids primarily responsible for antigen recognition. The carboxy-terminal portion of each chain defines a constant region primarily responsible for effector function.

Light chains are classified as kappa or lambda and characterized by a particular constant region. Heavy chains are classified as gamma, mu, alpha, delta, or epsilon, and define the antibody's isotype as IgG, IgM, IgA, IgD, and IgE, respectively. Each heavy chain type is characterized by a particular constant region.

Each heavy chain is comprised of a heavy chain variable region (herein "HCVR") and a heavy chain constant region. The heavy chain constant region is comprised of three domains (CH1, CH2, and CH3) for IgG, IgD, and IgA; and 4 domains (CH1, CH2, CH3, and CH4) for IgM and IgE. Each light chain is comprised of a light chain variable region (herein "LCVR") and a light chain constant region. The light chain constant region is comprised of one domain, CL. The HCVR and LCVR regions can be further subdivided into regions of hypervariability, termed complementarity determining regions (CDRs), interspersed with regions that are more conserved, termed framework regions (FR). Each HCVR and LCVR is composed of three CDRs and four FRs, arranged from amino-terminus to carboxy-terminus in the following order: FR1, CDR1, FR2, CDR2, FR3, CDR3, FR4. The assignment of amino acids to each domain is in accordance with well-known conventions [e.g., Kabat, "Sequences of Proteins of Immunological Interest," National Institutes of Health, Bethesda, Md. (1991) or Chothia numbering scheme as described in Al-Lazikani et al., J. Mol. Biol. 273:927-948, 1997, see also the internet site http:www.rubic.rdg.ac.uk/∼andrew/bioinf.orglabs. The functional ability of an antibody to bind a particular antigen is determined collectively by the six CDRs. However, even a single variable domain comprising only three CDRs specific for an antigen may have the ability to recognize and bind antigen, although at a lower affinity than a complete Fab.

The term "antibody," in reference to an anti-myostatin monoclonal antibody of the invention (or simply, "monoclonal antibody of the invention"), as used herein, refers to a monoclonal antibody. A "monoclonal antibody" as used herein refers to a rodent, preferably murine antibody, a chimeric antibody, a primatized antibody or a humanized antibody. Monoclonal antibodies of the invention can be produced using *e.g.*, hybridoma techniques well known in the art, as well as recombinant technologies, phage display technologies, synthetic technologies or combinations of such technologies readily known in the art. The term "monoclonal antibody" as used herein is not limited to antibodies produced through hybridoma technology. "Monoclonal antibody" refers to an antibody that is derived from a single copy or clone, including *e.g.*, any eukaryotic, prokaryotic, or phage clone, and not the method by which it is produced. A "monoclonal antibody" can be an intact (complete or full length) antibody, a substantially intact antibody, or a portion or fragment of an antibody comprising an antigen-binding portion, e.g., a Fab fragment, Fab' fragment or F(ab')₂ fragment of a murine antibody or of a chimeric antibody or of a humanized antibody.

As used herein, the "antigen-binding portion" or "antigen-binding region" or ""antigen-binding domain" refers interchangeably herein to that portion of an antibody molecule which contains the amino acid residues that interact with an antigen and confer on the antibody its specificity and affinity for the antigen. This antibody portion includes the "framework" amino acid residues necessary to maintain the proper conformation of the antigen-binding residues. Preferably, the CDRs of the antigen-binding region of the antibodies of the invention will be of murine origin. In other embodiments, the antigen-binding region can be derived from other non-human species including, but not limited to, rabbit, rat or hamster.

Furthermore, a "monoclonal antibody" as used herein can be a single chain Fv fragment that may be produced by joining the DNA encoding the LCVR and HCVR with a linker sequence. (See, Pluckthun, The Pharmacology of Monoclonal Antibodies, vol. 113, Rosenburg and Moore eds., Springer-Verlag, New York, pp 269-315, 1994). It is understood that regardless of whether fragments are specified, the term "antibody" as used herein includes such fragments as well as single chain forms. As long as the protein retains the ability to specifically or preferentially bind its intended target (*i.e.*, epitope or antigen), it is included within the term "antibody." Antibodies may or may not be glycosylated and still fall within the bounds of the invention.

A population of "monoclonal antibodies," refers to a homogeneous or substantially homogeneous (or pure) antibody population (*i.e.*, at least about 90%, 91%, 92%, 93%, 94%, 95%, 96%, more preferably at least about 97% or 98% or most preferably at least 99% of the antibodies in the population are identical and would compete in an ELISA assay for the same antigen or epitope.

The term "specifically binds" or "preferentially binds" as used herein refers to the situation in which one member of a specific binding pair does not significantly bind to molecules other than its specific binding partner(s). The term is also applicable where e.g., an antigen-binding domain of an antibody of the invention is specific for a particular epitope that is carried by a number of antigens, in which case the specific antibody carrying the antigen-binding domain will be able to bind to the various antigens carrying the epitope. Accordingly a monoclonal antibody of the invention specifically binds and/or preferentially binds myostatin while it does not specifically bind or preferentially bind GDF-11.

In one embodiment, a monoclonal antibody of the invention has less than about 20% cross-reactivity (more preferably, 19, 18, 17, 16, 15, 14, 13, 12, 11, 10, 9, 8, 7, 6, 5, 4, 3, 2 or 1 per cent cross-reactivity) with a non-myostatin protein or peptide (such as, *e.g.,* GDF11) or a protein that does not comprise 17, 16, 15, 14, 13, 12, 11, 10, 9, 8, 7, 6 or 5 contiguous amino acids of the sequence shown in SEQ ID NO: 46 or 43 as measured by a standard technique in the art such as an ELISA assay, a competitive ELISA assay or K_{D} values as measured in a BIAcore^{®} assay. Preferably an antibody of the invention binds myostatin at least 5, 10, 20, 30, 40, 50, 60, 70, 80, 90, or 100-fold greater than with which it binds GDF-11; more preferably at least 150, 200, 250, 300, 350, 400, 450, 500, 550 or 600-fold greater than with which it binds GDF-11, as determined e.g., by competition ELISA or BIAcore^{®} assay. Most preferably, the antibodies of the invention do not bind GDF-11 at levels greater than background levels of any binding assay available to the art. A monoclonal antibody of the invention may bind a monomeric or dimeric form of myostatin or a portion thereof.

The phrases "biological property" or "biological characteristic," or the terms "activity" or "bioactivity," in reference to an antibody of the present invention, are used interchangeably herein and include, but are not limited to, epitope/antigen affinity and specificity (e.g., anti-myostatin monoclonal antibody binding to myostatin or a peptide consisting of the sequence shown in SEQ ID NO: 46 or 43), ability to antagonize an activity of myostatin *in vivo*, *in vitro,* or *in situ*, the *in vivo* stability of the antibody and the immunogenic properties of the antibody. Other identifiable biological properties or characteristics of an antibody recognized in the art include, for example, cross-reactivity, (i.e., with non-human homologs of the targeted peptide, or with other proteins or tissues, generally), and ability to preserve high expression levels of protein in mammalian cells. The aforementioned properties or characteristics can be observed or measured or assessed using art-recognized techniques including, but not limited to, ELISA, competitive ELISA, BIAcore^{®} surface plasmon resonance analysis, *in vitro* and *in vivo* neutralization assays without limit, receptor binding, cytokine or growth factor production and/or secretion, Xenopus animal cap development, signal transduction and immunohistochemistry with tissue sections from different sources including human, primate, or any other source as the need may be.

The term "inhibit" or "neutralize" as used herein with respect to an activity of an antibody of the invention means the ability to substantially antagonize, prohibit, prevent, restrain, slow, disrupt, eliminate, stop, or reverse e.g., progression or severity of that which is being inhibited including, but not limited to, a biological activity or property, a disease or a condition.

The term "isolated" when used in relation to a nucleic acid or protein (e.g., an antibody) refers to a nucleic acid sequence or protein that is identified and separated from at least one contaminant with which it is ordinarily associated in its natural source. Preferably, an "isolated antibody" is an antibody that is substantially free of other antibodies having different antigenic specificities (e.g., pharmaceutical compositions of the invention comprise an isolated antibody that specifically binds myostatin and is substantially free of antibodies that specifically bind antigens other than myostatin).

The terms "Kabat numbering" and "Kabat labeling" are used interchangeably herein. These terms, which are recognized in the art, refer to a system of numbering amino acid residues which are more variable (i.e., hypervariable) than other amino acid residues in the heavy and light chain variable regions of an antibody (Kabat, et al., Ann. NY Acad. Sci. 190:382-93 (1971); Kabat, et al., Sequences of Proteins of Immunological Interest, Fifth Edition, U.S. Department of Health and Human Services, NIH Publication No. 91-3242 (1991)).

A polynucleotide is "operably linked" when it is placed into a functional relationship with another polynucleotide. For example, a promoter or enhancer is operably linked to a coding sequence if it affects the transcription of the sequence.

The terms "individual, " "subject," and "patient," used interchangeably herein, refer to a mammal, including, but not limited to, murines, simians, humans, mammalian farm animals, mammalian sport animals, and mammalian pets; preferably the term refers to humans.

The term "vector" includes a nucleic acid molecule capable of transporting another nucleic acid to which it has been linked including, but not limited to, plasmids and viral vectors. Certain vectors are capable of autonomous replication in a host cell into which they are introduced while other vectors can be integrated into the genome of a host cell upon introduction into the host cell, and thereby, are replicated along with the host genome. Moreover, certain vectors are capable of directing the expression of genes to which they are operably linked. Such vectors are referred to herein as "recombinant expression vectors" (or simply "expression vectors") and exemplary vectors are well known in the art.

The term "host cell" includes an individual cell or cell culture that is a recipient of any isolated polynucleotide of the invention or any recombinant vector(s) comprising a HCVR, LCVR or monoclonal antibody of the invention. Host cells include progeny of a single host cell, and the progeny may not necessarily be completely identical (in morphology or in total DNA complement) to the original parent cell due to natural, accidental, or deliberate mutation and/or change. A host cell includes cells transformed, transduced or infected *in vivo, in situ* or *in vitro* with a recombinant vector or a polynucleotide expressing a monoclonal antibody of the invention or a light chain or heavy chain thereof. A host cell which comprises a recombinant vector of the invention (either stably incorporated into the host chromosome or not) may also be referred to as a "recombinant host cell". Preferred host cells for use in the invention are CHO cells (*e.g*., ATCC CRL-9096), NS0 cells, SP2/0 cells and COS cells (ATCC *e.g.,* CRL-1650, CRL-1651), HeLa (ATCC CCL-2). Additional host cells for use in the invention include plant cells, yeast cells, other mammalian cells and prokaryotic cells.

The present invention relates to isolated, monoclonal antibodies that bind myostatin. Specifically, the antibodies of the invention bind the region of the mature form of myostatin spanning amino acids 40-64. Furthermore, antibodies of the invention neutralize a myostatin biological activity *in vivo*, *in vitro* or *in situ.* Specific binding of anti-myostatin monoclonal antibodies of the invention, (including antigen-binding portions thereof, and humanized monoclonal antibodies with like specificity) to myostatin allows said antibodies to be used as therapeutics or prophylactics for myostatin-associated diseases and disorders, i.e., diseases or disorders which benefit from lowering myostatin levels or inhibiting a myostatin biological activity.

The epitope to which the antibodies of the invention bind ("myostatin epitope of the invention") is localized within the peptide spanning amino acids 40 and 64 of mature myostatin of any mammalian species, preferably human. Antibodies which bind said epitope, specifically or preferentially bind myostatin when compared to their binding to GDF-11.

The term "epitope" refers to that portion of a molecule capable of being recognized by and bound by an antibody at one or more of the antibody's antigen-binding regions. Epitopes often consist of a chemically active surface grouping of molecules such as amino acids or sugar side chains and have specific three-dimensional structural characteristics as well as specific charge characteristics. By "inhibiting epitope" and/or "neutralizing epitope" is intended an epitope, which when in the context of the intact molecule (in this case, myostatin) and when bound by an antibody, results in loss or diminution of a biological activity of the molecule or organism containing the molecule, *in vivo*, *in vitro* or *in situ.*

The term "epitope," as used herein, further refers to a portion of a polypeptide having antigenic and/or immunogenic activity in an animal, preferably a mammal, e.g., a mouse or a human. The term "antigenic epitope," as used herein, is defined as a portion of a polypeptide to which an antibody can specifically bind as determined by any method well known in the art, for example, by conventional immunoassays. Antigenic epitopes need not necessarily be immunogenic, but may be immunogenic. An "immunogenic epitope," as used herein, is defined as a portion of a polypeptide that elicits an antibody response in an animal, as determine by any method known in the art. (See, *e.g*., Geysen et al., Proc. Natl. Acad. Sci. USA 81:3998-4002 (1983)). The human myostatin antigenic epitope of the present invention has the amino acid sequence as shown in SEQ ID NOs: 43 and 46. A myostatin antigenic epitope of the present invention for any mammalian species exists within a peptide consisting of amino acids 40-64 of the mature form of myostatin.

The anti-myostatin monoclonal antibodies of the invention bind an antigenic epitope discovered to be localized to amino acids 40 to 64 of mature myostatin. A myostatin immunogenic and/or antigenic epitope of the invention consists of a sequence as shown in SEQ ID NOs: 46 or 43, or consists of 24, 23, 22, 21, 20, 19, 18, 17, 16, 15, 14, 13, 12, 11, 10, 9, 8, 7, 6 or 5 contiguous amino acids of a peptide consisting of a sequence as shown in SEQ ID NOs: 46 or 43, preferably the immunogenic epitope spans amino acid residues in which 1, 2, 3, 4 or 5 of said contiguous amino acids are selected from the group consisting of amino acids at residue numbers 46, 49, 50, 52 and 62 of mature myostatin, i.e., where the amino acid at said residue number differs from the amino acid present at the equivalent position of GDF-11 (see Fig. 3). Furthermore, a myostatin immunogenic epitope of the invention is within positions 40-64 of the mature form of myostatin of any mammal or consists of 24, 23, 22, 21, 20, 19, 18, 17, 16, 15, 14, 13, 12, 11, 10, 9, 8, 7, 6 or 5 contiguous amino acids of a peptide consisting of the amino acids at positions 40-64 of the mature form of myostatin of any mammal, preferably said immunogenic epitope spans amino acid residues in which 1, 2, 3, 4 or 5 of said contiguous amino acids are amino acids which differ from the amino acid present at the equivalent position of GDF-11 in the same mammal. An immunogenic epitope of the invention is also contemplated to be an antigenic epitope. The antigenic epitope may possess additional myostatin residues outside of amino acids 40-64 of mature myostatin, but the monoclonal antibodies of the invention do not require these additional residues to specifically bind myostatin. Additionally, residues of myostatin outside of the amino acids 40-64 (i.e., the antigenic epitope) may affect the conformational structure of the antigenic domain and thereby alter binding of an antibody of the invention to the antigenic epitope. The monoclonal antibodies of the invention bind myostatin at least 5, 10, 20, 30, 40, 50, 60, 70, 80, 90, or 100-fold greater (e.g., greater affinity or greater specificity) than with which it binds GDF-11; more preferably at least 150, 200, 250, 300, 350, 400, 450, 500, 550 or 600-fold greater than with which it binds GDF-11, as determined e.g., by ELISA assay, competition ELISA assay or K_{D} values in a Biacore® assay.

The domain spanning amino acids 40-64 (inclusive) of mature myostatin or any peptide consisting of an immunogenic epitope as described herein may be used as an immunogenic peptide, preferably conjugated to a carrier protein e.g., KLH, to generate monoclonal antibodies of the invention. The immunogenic peptide may be used to immunize a non-human animal, preferably a mammal, more preferably a mouse. Then anti-myostatin antibodies are isolated from the immunized animal and screened by methods well known in the art to isolate those antibodies that specifically bind amino acids 40-64 of myostatin.

Generally, a hybridoma can be produced by fusing a suitable immortal cell line (e.g., a myeloma cell line such as SP2/0) with antibody producing cells of the immunized animal. The antibody producing cell, preferably those of the spleen or lymph nodes, are obtained from animals immunized with the antigen of interest. The fused cells (hybridomas) can be isolated using selective culture conditions, and cloned by limiting dilution. Cells which produce antibodies with the desired binding properties can be selected by a suitable assay. Methods for such isolation and screening are well known in the art. Selection of antibody fragments from libraries using enrichment technologies such as phage-display (Matthews DJ and Wells JA. Science. 260:1113-7, 1993), ribosome display (Hanes, et al., Proc. Natl. Acad. Sci. (USA) 95:14130-5, 1998), bacterial display (Samuelson P., et al., Journal of Biotechnology. 96:129-54, 2002) or yeast display (Kieke MC, et al., Protein Engineering, 10:1303-10, 1997) has proven to be successful alternatives to classical hybridoma technology (recent reviews: Little M. et al., Immunology Today, 21:364-70, 2000;). Antibodies of the invention may be altered to a chimeric or humanized form using methods well known in the art.

Other suitable methods of producing or isolating antibodies which bind amino acids 40-64 of mature myostatin, including human or artificial antibodies, can be used, including, for example, methods which select a recombinant antibody (*e.g*., single chain Fv or Fab) from a library, or which rely upon immunization of transgenic animals (*e.g*., mice) capable of producing a repertoire of human antibodies (see e.g., Jakobovits et al., Proc. Natl. Acad. Sci. USA, 90:2551-2555, 1993; Jakobovits et al., Nature, 362:255-258, 1993; Lonberg et al., U.S. Patent Number 5,545,806; Surani et al., U.S. Patent Number 5,545,807).

Single chain antibodies, and chimeric, humanized or primatized (CDR-grafted) antibodies, as well as chimeric or CDR-grafted single chain antibodies, and the like, comprising portions derived from different species, are also encompassed by the present invention and the term "antibody". The various portions of these antibodies can be joined together chemically by conventional techniques, synthetically, or can be prepared as a contiguous protein using genetic engineering techniques. For example, nucleic acids encoding a chimeric or humanized chain can be expressed to produce a contiguous protein. See e.g., U.S. Patent No. 4,816,567; European Patent No. 0,125,023 B1; U.S. Patent No. 4,816,397; European Patent No. 0,120,694 B1; WO 86/01533; European Patent No. 0,194,276 B1; U.S. Patent No. 5,225, 539; European Patent No. 0,239,400 B1 and U.S. Patent Nos. 5,585,089 and 5,698,762. See also, Newman, R. et al. BioTechnology, 10:1455-1460, 1993, regarding primatized antibody, and Ladner et al., U.S. Patent No. 4,946,778 and Bird, R.E. et al., Science, 242:423-426, 1988, regarding single chain antibodies.

In addition, functional fragments of antibodies, including fragments of chimeric, humanized, primatized or single chain antibodies, can also be produced. Functional fragments of the foregoing antibodies retain at least one binding function and/or biological function of the full-length antibody from which they are derived. Preferred functional fragments retain an antigen-binding function of a corresponding full-length antibody (e.g., the ability to bind a mammalian mature form of myostatin). Particularly preferred functional fragments retain the ability to inhibit one or more functions or bioactivities characteristic of a mammalian mature myostatin, such as a binding activity, a signaling activity, and/or stimulation of a cellular response. For example, in one embodiment, a functional fragment can inhibit the interaction of mature myostatin with one or more of its ligands and/or can inhibit one or more receptor-mediated functions.

Antibody fragments capable of binding to a mammalian mature myostatin or portion thereof, include, but are not limited to, Fv, Fab, Fab' and F(ab')₂ fragments are encompassed by the invention. Such fragments can be produced by enzymatic cleavage or by recombinant techniques. For instance, papain or pepsin cleavage can generate Fab or F(ab')₂ fragments, respectively. Antibodies can also be produced in a variety of truncated forms using antibody genes in which one or more stop codons has been introduced upstream of the natural stop site. For example, a chimeric gene encoding a F(ab')₂ heavy chain portion can be designed to include DNA sequences encoding the CH₁ domain and hinge region of the heavy chain.

In a preferred embodiment, the invention provides an anti-myostatin monoclonal antibody resulting from the process described that preferably binds mature myostatin or a portion thereof with an affinity of at least about 1 x 10⁻⁷ M, preferably at least about 9 x 10⁻⁸ M or 7 x 10⁻⁸ M, and more preferably at least about 5 x 10⁻⁸ M. (as determined e.g., by solid phase BIAcore^{®} surface plasmon resonance assay) and has the capacity to antagonize a biological activity of a mature myostatin.

A preferred monoclonal antibody of the invention has a LCVR comprising a peptide with a sequence selected from the group consisting of SEQ ID NOs: 3, 4, 5, 6, 7,8, 9, 10 and 11 and/or a HCVR comprising a peptide with a sequence selected from the group consisting of SEQ ID NOs: 12, 13, 14, 15, 16, 17, and SEQ ID NO: 12 with amino acids 26-37 replaced with the amino acids in SEQ ID NO: 47, 48, 49, 50, 51, 52, 53 or 54. (See Tables 1 and 2; and Figs 4 and 5 herein for sequences and their locations in the Fabs). Furthermore, a monoclonal antibody of the invention is one that is competitively inhibited from binding mature human myostatin (or a portion thereof) by a monoclonal antibody comprising two polypeptides with the sequences shown in the group consisting of (i) SEQ ID NOs: 3 and 12, (ii) SEQ ID NOs: 4 and 13, (iii) SEQ ID NOs: 3 and 14, (iv) SEQ ID NOs: 5 and 12, (v) SEQ ID NOs: 6 and 15, (vi) SEQ ID NOs: 7 and 17, (vii) SEQ ID NOs: 8 and 12, (viii) SEQ ID NOs: 9 and 16, (ix) SEQ ID NOs: 10 and 12, and (x) SEQ ID NOs: 11 and 12, and (xi) SEQ ID NO: 3 and SEQ ID NO: 12 with amino acids 26-37 replaced with the amino acids in SEQ ID NO: 47, 48, 49, 50, 51, 52, 53 or 54.

In another embodiment, a LCVR of an anti-myostatin monoclonal antibody of the invention comprises 1, 2 or 3 peptides selected from the group consisting of peptides with a sequence as shown in SEQ ID NOs: 38, 23 and 56 (see Table 1). A HCVR of an anti-myostatin monoclonal antibody of the invention comprises 1, 2 or 3 peptides selected from the group consisting of peptides with a sequence as shown in SEQ ID NOs: 55, 41 and 42 (see Table 2).

In a preferred embodiment, an anti-myostatin monoclonal antibody of the invention is a chimeric antibody or a humanized antibody. Alternatively, the framework and any constant region present in the antibody may substantially originate from the genome of the animal in which the antibody is to be used as a therapeutic. A preferred antibody is a full-length antibody.

The present invention is also directed to cell lines that express an anti-myostatin monoclonal antibody of the invention or portion thereof. Creation and isolation of cell lines producing a monoclonal antibody of the invention can be accomplished using standard techniques known in the art. Preferred cell lines include COS, CHO, SP2/0, NS0 and yeast (available from public repositories such as ATCC, American Type Culture Collection, Manassas, VA).

A wide variety of host expression systems can be used to express an antibody of the present invention including prokaryotic (bacterial) and eukaryotic expression systems (such as yeast, baculovirus, plant, mammalian and other animal cells, transgenic animals, and hybridoma cells), as well as phage display expression systems. An example of a suitable bacterial expression vector is pUC 119 and a suitable eukaryotic expression vector is a modified pcDNA3.1 vector with a weakened DHFR selection system. Other antibody expression systems are also known in the art and are contemplated herein.

An antibody of the invention can be prepared by recombinant expression of immunoglobulin light and heavy chain genes in a host cell. To express an antibody recombinantly, a host cell is transformed, transduced, infected or the like with one or more recombinant expression vectors carrying DNA fragments encoding the immunoglobulin light and/or heavy chains of the antibody such that the light and/or heavy chains are expressed in the host cell. The heavy chain and the light chain may be expressed independently from different promoters to which they are operably linked in one vector or, alternatively, the heavy chain and the light chain may be expressed independently from different promoters to which they are operably linked in two vectors - one expressing the heavy chain and one expressing the light chain. Optionally the heavy chain and light chain may be expressed in different host cells. Preferably, the recombinant antibodies are secreted into the medium in which the host cells are cultured, from which the antibodies can be recovered or purified. Standard recombinant DNA methodologies are used to obtain antibody heavy and light chain genes, incorporate these genes into recombinant expression vectors, and introduce the vectors into host cells. Such standard recombinant DNA technologies are described, for example, in Sambrook, Fritsch, and Maniatis (Eds.), Molecular Cloning; A Laboratory Manual, Second Edition, Cold Spring Harbor, N.Y., 1989; Ausubel, et al (Eds.) Current Protocols in Molecular Biology, Greene Publishing Associates, 1989.

An isolated DNA encoding a HCVR region can be converted to a full-length heavy chain gene by operably linking the HCVR-encoding DNA to another DNA molecule encoding heavy chain constant regions (CH1, CH2, and CH3). The sequences of human heavy chain constant region genes are known in the art. See, *e.g*., Kabat, et al., Sequences of Proteins of Immunological Interest, Fifth Edition, U.S. Department of Health and Human Services, NIH Publication No. 91-3242 (1991). DNA fragments encompassing these regions can be obtained e.g., by standard PCR amplification. The heavy chain constant region can be of any type, (*e.g*., IgG, IgA, IgE, IgM or IgD), class (*e.g*., IgG₁, IgG₂, IgG₃ and IgG₄) or subclass constant region and any allotypic variant thereof as described in Kabat (*supra*). Alternatively, the antigen binding portion can be a Fab fragment, Fab' fragment, F(ab')₂ fragment, Fd, or a single chain Fv fragment (scFv). For a Fab fragment heavy chain gene, the HCVR-encoding DNA may be operably linked to another DNA molecule encoding only a heavy chain CH1 constant region.

An isolated DNA encoding a LCVR region may be converted to a full-length light chain gene (as well as a Fab light chain gene) by operably linking the LCVR-encoding DNA to another DNA molecule encoding a light chain constant region, CL. The sequences of human light chain constant region genes are known in the art. See, *e.g*., Kabat, *supra.* DNA fragments encompassing these regions can be obtained by standard PCR amplification. The light chain constant region can be a kappa or lambda constant region.

To create an scFv gene, the HCVR- and LCVR-encoding DNA fragments are operably linked to another fragment encoding a flexible linker, e.g., encoding the amino acid sequence (Gly₄-Ser)₃, such that the HCVR and LCVR sequences can be expressed as a contiguous single-chain protein, with the LCVR and HCVR regions joined by the flexible linker. See, *e.g.,* Bird, et al., Science 242:423-6, 1988; Huston, et al., Proc. Natl. Acad. Sci. USA 85:5879-83, 1988; McCafferty, et al., Nature 348:552-4, 1990.

To express an antibody of the invention, a DNA encoding a partial or full-length light and/or heavy chain, obtained as described above, are inserted into an expression vector such that the gene is operably linked to transcriptional and translational control sequences. The expression vector and expression control sequences are chosen to be compatible with the expression host cell used. The antibody light chain gene and the antibody heavy chain gene can be inserted into separate vectors or, more typically, both genes are inserted into the same expression vector. The antibody genes are inserted into the expression vector by standard methods. Additionally, the recombinant expression vector can encode a signal peptide that facilitates secretion of the anti-myostatin monoclonal antibody light and/or heavy chain from a host cell. The anti-myostatin monoclonal antibody light and/or heavy chain gene can be cloned into the vector such that the signal peptide is operably linked in-frame to the amino terminus of the antibody chain gene. The signal peptide can be an immunoglobulin signal peptide or a heterologous signal peptide.

In addition to the antibody heavy and/or light chain gene(s), a recombinant expression vector of the invention carries regulatory sequences that control the expression of the antibody chain gene(s) in a host cell. The term "regulatory sequence" is intended to include promoters, enhancers and other expression control elements (e.g., polyadenylation signals), as needed, that control the transcription or translation of the antibody chain gene(s). The design of the expression vector, including the selection of regulatory sequences may depend on such factors as the choice of the host cell to be transformed, the level of expression of protein desired. Preferred regulatory sequences for mammalian host cell expression include viral elements that direct high levels of protein expression in mammalian cells, such as promoters and/or enhancers derived from cytomegalovirus (CMV), Simian Virus 40 (SV40), adenovirus, (e.g., the adenovirus major late promoter (AdMLP)) and polyoma virus.

In addition to the antibody heavy and/or light chain genes and regulatory sequences, the recombinant expression vectors of the invention may carry additional sequences, such as sequences that regulate replication of the vector in host cells (e.g., origins of replication) and one or more selectable marker genes. The selectable marker gene facilitates selection of host cells into which the vector has been introduced. For example, typically the selectable marker gene confers resistance to drugs, such as G418, hygromycin, or methotrexate, on a host cell into which the vector has been introduced. Preferred selectable marker genes include the dihydrofolate reductase (DHFR) gene (for use in DHFR-minus host cells with methotrexate selection/amplification), the *neo* gene (for G418 selection), and glutamine synthetase (GS) in a GS-negative cell line (such as NS0) for selection/amplification.

For expression of the light and/or heavy chains, the expression vector(s) encoding the heavy and/or light chains is introduced into a host cell by standard techniques *e.g*., electroporation, calcium phosphate precipitation, DEAE-dextran transfection, transduction, infection and the like. Although it is theoretically possible to express the antibodies of the invention in either prokaryotic or eukaryotic host cells, preferably eukaryotic cells, and most preferably mammalian host cells, because such cells, are more likely to assemble and secrete a properly folded and immunologically active antibody. Preferred mammalian host cells for expressing the recombinant antibodies of the invention include Chinese Hamster Ovary (CHO cells) (including DHFR-CHO cells, described in Urlaub and Chasin, Proc. Natl. Acad. Sci. USA 77:4216-20, 1980, used with a DHFR selectable marker, e.g., as described in Kaufman and Sharp, J. Mol. Biol. 159:601-21, 1982, NS0 myeloma cells, COS cells, and SP2/0 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the host cell and/or the culture medium using standard purification methods.

Host cells can also be used to produce portions, or fragments, of intact antibodies, *e.g.,* Fab fragments or scFv molecules by techniques that are conventional *per se.* It will be understood that variations on the above procedure are within the scope of the present invention. For example, it may be desirable to transfect a host cell with DNA encoding either the light chain or the heavy chain of an antibody of this invention. Recombinant DNA technology may also be used to remove some or all the DNA encoding either or both of the light and heavy chains that is not necessary for binding to myostatin. The molecules expressed from such truncated DNA molecules are also encompassed by the antibodies of the invention.

In a preferred system for recombinant expression of an antibody of the invention, a recombinant expression vector encoding both the antibody heavy chain and the antibody light chain is introduced into DHFR-CHO cells by e.g., calcium phosphate-mediated transfection. Within the recombinant expression vector, the antibody heavy and light chain genes are each operably linked to enhancer/promoter regulatory elements (*e.g*., derived from SV40, CMV, adenovirus and the like, such as a CMV enhancer/AdMLP promoter regulatory element or an SV40 enhancer/AdMLP promoter regulatory element) to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium. Antibodies, or antigen-binding portions thereof, of the invention can be expressed in an animal (*e.g*., a mouse) that is transgenic for human immunoglobulin genes (see, *e.g*., Taylor, et al., Nucleic Acids Res. 20:6287-95, 1992).

Once expressed, the intact antibodies, their dimers, individual light and heavy chains, or other immunoglobulin forms of the present invention can be purified according to standard procedures of the art, including ammonium sulfate precipitation, ion exchange, affinity, reverse phase, hydrophobic interaction column chromatography, gel electrophoresis and the like. Substantially pure immunoglobulins of at least about 90%, 92%, 94% or 96% homogeneity are preferred, and 98 to 99% or more homogeneity most preferred, for pharmaceutical uses. Once purified, partially or to homogeneity as desired, the peptides may then be used therapeutically or prophylactically, as directed herein.

As used herein, the term "chimeric antibody" includes monovalent, divalent or polyvalent immunoglobulins. A monovalent chimeric antibody is a dimer formed by a chimeric heavy chain associated through disulfide bridges with a chimeric light chain. A divalent chimeric antibody is a tetramer formed by two heavy chain-light chain dimers associated through at least one disulfide bridge.

A chimeric heavy chain of an antibody for use in humans comprises an antigen-binding region derived from the heavy chain of a non-human antibody specific for myostatin, which is linked to at least a portion of a human heavy chain constant region, such as CH1 or CH2. A chimeric light chain of an antibody for use in humans comprises an antigen binding region derived from the light chain of a non-human antibody specific for myostatin, linked to at least a portion of a human light chain constant region (CL). Antibodies, fragments or derivatives having chimeric heavy chains and light chains of the same or different variable region binding specificity, can also be prepared by appropriate association of the individual polypeptide chains, according to known method steps.

With this approach, hosts expressing chimeric heavy chains are separately cultured from hosts expressing chimeric light chains, and the immunoglobulin chains are separately recovered and then associated. Alternatively, the hosts can be co-cultured and the chains allowed to associate spontaneously in the culture medium, followed by recovery of the assembled immunoglobulin or fragment.

Methods for producing chimeric antibodies are known in the art (see, e.g., U.S. Patent NOs.: 6,284,471; 5,807,715; 4,816,567; and 4,816,397).

In a preferred embodiment, a gene is created which comprises a first DNA segment that encodes at least the antigen-binding region of non-human origin (e.g., that of Fabs 3, 5, 7, 8, 9, 10, 11, 12, 14 or 15 as in Table 1, 2 and Figs 4 and 5 herein), such as functionally rearranged variable (V) region with joining (J) segment, linked to a second DNA segment encoding at least a part of a human constant (C) region as described in U.S. Patent No. 6,284,471.

Preferably an antibody of the invention to be used for therapeutic purposes, would have the sequence of the framework and constant region as exists in the antibody derived from the mammal in which it would be used as a therapeutic so as to decrease the possibility that the mammal would illicit an immune response against the therapeutic antibody.

Humanized antibodies are of particular interest, since they are considered to be valuable for therapeutic application, avoiding the human anti-mouse antibody response frequently observed with rodent antibodies. The term "humanized antibody" as used herein refers to an immunogloulin comprising portions of antibodies of different origin, wherein at least one portion is of human origin. For example, the humanized antibody can comprise portions derived from an antibody of nonhuman origin with the requisite specificity, such as a mouse, and from an antibody of human origin, joined together chemically by conventional techniques (e.g., synthetic) or prepared as a contiguous polypeptide using genetic engineering techniques. Preferably, a "humanized antibody" has CDRs that originate from a non-human antibody (preferably a mouse monoclonal antibody) while framework and constant region, to the extent it is present, (or a significant or substantial portion thereof, *i.e*., at least about 90%, 92%, 94%, 96%, 98% or 99%) are encoded by nucleic acid sequence information that occurs in the human germline immunoglobulin region (see, e.g., the International ImMunoGeneTics Database) or in recombined or mutated forms thereof whether or not said antibodies are produced in human cell. A humanized antibody may be an intact antibody, a substantially intact antibody, a portion of an antibody comprising an antigen-binding site, or a portion of an antibody comprising a Fab fragment, Fab' fragment, F(ab')₂, or a single chain Fv fragment. It is contemplated that in the process of creating a humanized antibody, the amino acid at either termini of a CDR (see e.g., Tables 1 and 2) may be substituted with an amino acid that occurs in the human germline for that segment of adjoining framework sequence.

Humanized antibodies may be subjected to *in vitro* mutagenesis using methods of routine use in the art (or, when an animal transgenic for human Ig sequences is used, *in vivo* somatic mutagenesis) and, thus, the framework region amino acid sequences of the HCVR and LCVR regions of the humanized recombinant antibodies are sequences that, while derived from those related to human germline HCVR and LCVR sequences, may not naturally exist within the human antibody germline repertoire *in vivo.* It is contemplated that such amino acid sequences of the HCVR and LCVR framework regions of the humanized recombinant antibodies are at least 90%, 92%, 94%, 96%, 98% or most preferably at least 99% identical to a human germline sequence.

Humanized antibodies have at least three potential advantages over non-human and chimeric antibodies for use in human therapy: (i) the effector portion is human, it may interact better with the other parts of the human immune system (e.g., destroy the target cells more efficiently by complement-dependent cytotoxicity or antibody-dependent cellular cytotoxicity); (ii) the human immune system should not recognize the framework or constant region of the humanized antibody as foreign, and therefore the antibody response against such an injected antibody should be less than that against a totally foreign non-human antibody or a partially foreign chimeric antibody; and (iii) injected non-human antibodies have been reported to have a half-life in the human circulation much shorter than the half-life of human antibodies. Injected humanized antibodies may have a half-life much like that of naturally occurring human antibodies, thereby allowing smaller and less frequent doses to be given.

Humanization may in some instances adversely affect antigen binding of the antibody. Preferably a humanized anti-myostatin monoclonal antibody of the present invention will possess a binding affinity for myostatin of not less than about 50%, more preferably not less than about 30%, and most preferably not less than about 25%, 20%, 15%, 10% or 5% of the binding affinity of the parent murine antibody, preferably Fab 3, 5, 7, 8, 9, 10, 11, 12, 14 or 15 for myostatin (see Figs 4 and 5 herein). Preferably, a humanized antibody of the present invention will bind the same epitope as does Fab 3, 5, 7, 8, 9, 10, 11, 12, 14 or 15 described herein. Said antibody can be identified based on its ability to compete with Fabs 3, 5, 7, 8, 9, 10, 11, 12, 14 or 15 for binding to mature myostatin or a peptide with the sequence as shown in SEQ ID NOs 46 or 43.

In general, the humanized antibodies are produced by obtaining nucleic acid sequences encoding the HCVR and LCVR of an antibody which binds a myostatin epitope of the invention, identifying the CDRs in said HCVR and LCVR (nonhuman), and grafting such CDR-encoding nucleic acid sequences onto selected human framework-encoding nucleic acid sequences. Preferably, the human framework amino acid sequences are selected such that the resulting antibody is likely to be suitable for *in vivo* administration in humans. This can be determined, *e.g*., based on previous usage of antibodies containing such human framework sequence. Preferably, the human framework sequence will not itself be significantly immunogenic.

Alternatively, the amino acid sequences of the frameworks for the antibody to be humanized (e.g., Fabs 3, 5, 7, 8, 9, 10, 11, 12, 14 or 15) will be compared to those of known human framework sequences the human framework sequences to be used for CDR-grafting will be selected based on their comprising sequences highly similar to those of the parent antibody, e.g., a murine antibody which binds myostatin. Numerous human framework sequences have been isolated and their sequences reported in the art. This enhances the likelihood that the resultant CDR-grafted humanized antibody, which contains CDRs of the parent (*e.g*., murine) antibody grafted onto selected human frameworks (and possibly also the human constant region) will substantially retain the antigen binding structure and thus retain the binding affinity of the parent antibody. To retain a significant degree of antigen binding affinity, the selected human framework regions will preferably be those that are expected to be suitable for *in vivo* administration, *i.e*., not immunogenic.

In either method, the DNA sequence encoding the HCVR and LCVR regions of the preferably murine anti-myostatin antibody are obtained. Methods for cloning nucleic acid sequences encoding immunoglobulins are well known in the art. Such methods may, for example, involve the amplification of the immunoglobulin-encoding sequences to be cloned using appropriate primers by polymerase chain reaction (PCR). Primers suitable for amplifying immunoglobulin nucleic acid sequences, and specifically murine HCVR and LCVR sequences have been reported in the literature. After such immunoglobulin-encoding sequences have been cloned, they will be sequences by methods well known in the art.

Once the DNA sequences encoding the CDRs and frameworks of the antibody which is to be humanized have been identified, the amino acid sequences encoding the CDRs are then identified (deduced based on the nucleic acid sequences and the genetic code and by comparison to previous antibody sequences) and the CDR-encoding nucleic acid sequences are grafted onto selected human framework-encoding sequences. This may be accomplished by use of appropriate primers and linkers. Methods for selecting suitable primers and linkers to prime for ligation of desired nucleic acid sequences is well within the ability of one of ordinary skill in the art.

After the CDR-encoding sequences are grafted onto the selected human framework encoding sequences, the resultant DNA sequences encoding the "humanized" variable heavy and variable light sequences are then expressed to produce a humanized Fv or humanized antibody that binds myostatin. Typically, the humanized HCVR and LCVR are expressed as part of a whole anti-myostatin antibody molecule, i*.e.*, as a fusion protein with human constant domain sequences whose encoding DNA sequences have been obtained from a commercially available library or which have been obtained using, e.g., one of the above described methods for obtaining DNA sequences, or are in the art. However, the HCVR and LCVR sequences can also be expressed in the absence of constant sequences to produce a humanized anti-myostatin Fv. Nevertheless, fusion of human constant sequences is potentially desirable because the resultant humanized anti-myostatin antibody may possess human effector functions.

Methods for synthesizing DNA encoding a protein of known sequence are well known in the art. Using such methods, DNA sequences which encode the subject humanized HCVR and LCVR sequences (with or without constant regions) are synthesized, and then expressed in a vector system suitable for expression of recombinant antibodies. This may be effected in any vector system which provides for the subject humanized HCVR and LCVR sequences to be expressed as a fusion protein with human constant domain sequences and to associate to produce functional (antigen binding) antibodies or antibody fragments.

Human constant domain sequences are well known in the art, and have been reported in the literature. Preferred human constant light chain sequences include the kappa and lambda constant light chain sequences. Preferred human constant heavy chain sequences include human gamma 1, human gamma 2, human gamma 3, human gamma r, and mutated versions thereof which provide for altered effect or function, e.g., enhanced *in vivo* half-life, reduced Fc receptor binding, and the like.

If present, human framework regions are preferably derived from a human antibody variable region having sequence similarity to the analogous or equivalent region of the antigen binding region donor. Other sources of framework regions for portions of human origin of a humanized antibody include human variable consensus sequences (see *e.g*., Kettleborough, C.A. et al. Protein Engineering 4:773-783 (1991); Carter et al., WO 94/04679. For example, the sequence of the antibody or variable region used to obtain the nonhuman portion can be compared to human sequences as described in Kabat et al. Sequences of Proteins of Immunological Interest, Fifth Edition, NIH, U.S. Government Printing Office (1991). In a particularly preferred embodiment, the framework regions of a humanized antibody chain are derived from a human variable region having at least about 60% overall sequence identity, preferably at least about 70% overall sequence identity and more preferably at least about 85% overall sequence identity, with the variable region of the nonhuman donor. A human portion can also be derived from a human antibody having at least about 65% sequence identity, and preferably at least about 70% sequence identity, within the particular portion (*e.g*., FR) being used, when compared to the equivalent portion (*e.g*., FR) of the nonhuman donor.

In some instances, humanized antibodies produced by grafting CDRs (from an antibody which binds myostatin) onto selected human frameworks may provide humanized antibodies having the desired affinity to myostatin. However, it may be necessary or desirable to further modify specific residues of the selected human framework in order to enhance antigen binding. Preferably, those framework residues of the parent (*e.g*., murine) antibody which maintain or affect combining-site structures will be retained. These residues may be identified by X-ray crystallography of the parent antibody or Fab fragment, thereby identifying the three-dimensional structure of the antigen-binding site.

References further describing methods involved in humanizing a mouse antibody that may be used are *e.g*., Queen et al., Proc. Natl. Acad. Sci. USA 88:2869, 1991; U.S. Pat. No. 5,693,761; U.S. Pat. No. 4,816,397; U.S. Pat. No. 5,225,539; computer programs ABMOD and ENCAD as described in Levitt, M., J. Mol. Biol. 168:595-620, 1983.

Antibodies of the present invention are useful in therapeutic, diagnostic and research applications as described herein. An antibody of the invention may be used to diagnose a disorder or disease associated with the expression of human myostatin. In a similar manner, the antibody of the invention can be used in an assay to monitor myostatin levels in a subject being treated for a myostatin-associated condition. Diagnostic assays include methods that utilize the antibody of the invention and a label to detect myostatin in a sample, *e.g*., in a human body fluid or in a cell or tissue extract. Binding compositions, such as, *e.g*., antibodies, are used with or without modification, and are labeled by covalent or non-covalent attachment of a detectable moiety. The detectable moiety can be any one that is capable of producing, either directly or indirectly, a detectable signal. For example, the detectable moiety may be a radioisotope such as, e.g., ³H, ¹⁴C, ³²P, ³⁵S or ¹²⁵I, a fluorescent or chemiluminescent compound, such as fluorescein isothiocyanate, rhodamine, or luciferin; or an exzyme, such as alkaline phosphatase, beta-galactosidase, or horseradish peroxidase. Any method known in the art for separately conjugating the antibody to the detectable moiety may be employed, including those methods described by Hunter, et al., Nature 144:945, 1962; David, et al., Biochemistry 13: 1014, 1974; Pain, et al., J. Immunol. Meth. 40: 219, 1981; and Nygren, J. Histochem. And Cytochem. 30: 407, 1982.

A variety of conventional protocols for measuring myostatin, including *e.g*., ELISA, RIAs, and FACS, are known in the art and provide a basis for diagnosing altered or abnormal levels of myostatin expression. Normal or standard expression values are established using any art known technique, *e.g*., by combining a sample comprising a myostatin polypeptide with, *e.g*., antibodies under conditions suitable to form a antigen:antibody complex. The antibody is directly or indirectly labeled with a detectable substance to facilitate detection of the bound or unbound antibody. Suitable detectable substances include various enzymes, prosthetic groups, fluorescent materials, luminescent materials and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; and examples of a radioactive material include ¹²⁵I, ¹³¹I, ³⁵S, or ³H. (See, *e.g*., Zola, Monoclonal Antibodies: A Manual of Techniques, CRC Press, Inc. (1987)).

The amount of a standard complex formed is quantitated by various methods, such as, *e.g*., photometric means. Amounts of myostatin polypeptide expressed in subject, control, and samples (*e.g*., from biopsied tissue) are then compared with the standard values. Deviation between standard and subject values establishes parameters for correlating a particular disorder, state, condition, syndrome, or disease with a certain level of expression (or lack thereof) for a myostatin polypeptide.

Once the presence of a disorder, state, condition, syndrome, or disease is established and a treatment protocol is initiated, assays are repeated on a regular basis to monitor the level of myostatin expression. The results obtained from successive assays are used to show the efficacy of treatment over a period ranging from several days to months. With respect to a particular disorders (*e.g*., frailty or cachexia) the presence of an altered amount of myostatin in biopsied tissue or fluid (*e.g*., serum or urine) from a subject may indicate a predisposition for the development of a disorder, state, condition, syndrome, or disease or it may provide a means for detecting such a disorder, state, condition, syndrome, or disease prior to the appearance of actual clinical symptoms or it may define a population more likely to respond therapeutically to an antibody of the invention. A more definitive initial detection may allow earlier treatment thereby preventing and/or ameliorating further progression of cell proliferation.

An antibody of the invention can be incorporated into pharmaceutical compositions suitable for administration to a subject. The compounds of the invention may be administered alone or in combination with a pharmaceutically acceptable carrier, diluent, and/or excipients, in single or multiple doses. The pharmaceutical compositions for administration are designed to be appropriate for the selected mode of administration, and pharmaceutically acceptable diluents, carrier, and/or excipients such as dispersing agents, buffers, surfactants, preservatives, solubilizing agents, isotonicity agents, stabilizing agents and the like are used as appropriate. Said compositions are designed in accordance with conventional techniques as in *e.g.,* Remington, The Science and Practice of Pharmacy, 19th Edition, Gennaro, Ed., Mack Publishing Co., Easton, PA 1995 which provides a compendium of formulation techniques as are generally known to practitioners.

A pharmaceutical composition comprising an anti-myostatin monoclonal antibody of the present invention can be administered to a subject at risk for or exhibiting pathologies as described herein using standard administration techniques including oral, intravenous, intraperitoneal, subcutaneous, pulmonary, transdermal, intramuscular, intranasal, buccal, sublingual, or suppository administration.

A pharmaceutical composition of the invention preferably is a "therapeutically effective amount" or a "prophylactically effective amount" of an antibody of the invention. A "therapeutically effective amount" refers to an amount effective, at dosages and for periods of time necessary, to achieve the desired therapeutic result. A therapeutically effective amount of the antibody may vary according to factors such as the disease state, age, sex, and weight of the individual, and the ability of the antibody or antibody portion to elicit a desired response in the individual. A therapeutically effective amount is also one in which any toxic or detrimental effect of the antibody, are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

A therapeutically-effective amount is at least the minimal dose, but less than a toxic dose, of an active agent which is necessary to impart therapeutic benefit to a subject. Stated another way, a therapeutically-effective amount is an amount which in mammals, preferably humans, increases muscle mass, increases bone density, or treats conditions wherein the presence of myostatin causes or contributes to undesirable pathological effects or decrease in myostatin levels results in a beneficial therapeutic effect in a mammal, preferably a human, including, but not limited to, muscle wasting, frailty, age-related sarcopenia, osteoporosis, obesity, muscular dystrophy of any type, critical care myopaythy, sepsis, cachexia (*e.g*., cancer-related or HIV-induced), COPD, osteoarthritis, renal failure, liver failure, cardiac failure or disease, metabolic syndrome and Type II diabetes.

The route of administration of an antibody of the present invention may be oral, parenteral, by inhalation, or topical. Preferably, the antibodies of the invention can be incorporated into a pharmaceutical composition suitable for parenteral administration. The term parenteral as used herein includes intravenous, intramuscular, subcutaneous, rectal, vaginal, or intraperitoneal administration. Peripheral systemic delivery by intravenous or intraperitoneal or subcutaneous injection is preferred. Suitable vehicles for such injections are straightforward in the art.

The pharmaceutical composition typically must be sterile and stable under the conditions of manufacture and storage in the container provided, including *e.g*., a sealed vial or syringe. Therefore, pharmaceutical compositions may be sterile filtered after making the formulation, or otherwise made microbiologically acceptable. A typical composition for intravenous infusion could have a volume as much as 250-1000 ml of fluid, such as sterile Ringer's solution, physiological saline, dextrose solution and Hank's solution and a therapeutically effective dose, (*e.g*., 1 to 100 mg/mL, or more) of antibody concentration. Dose may vary depending on the type and severity of the disease. As is well known in the medical arts, dosages for any one subject depends upon many factors, including the patient's size, body surface area, age, the particular compound to be administered, sex, time and route of administration, general health, and other drugs being administered concurrently. A typical dose can be, for example, in the range of 0.001 to 1000 µg; however, doses below or above this exemplary range are envisioned, especially considering the aforementioned factors. The daily parenteral dosage regimen can be about 0.1 µg/kg to about 100 mg/kg of total body weight, preferably from about 0.3 µg/kg to about 10 mg/kg and more preferably from about 1 µg/kg to 1 mg/kg, even more preferably from about 0.5 to 10 mg/kg body weight per day. Progress may be monitored by periodic assessment. For repeated administrations over several days or longer, depending on the condition, the treatment is repeated until a desired suppression of disease symptoms occurs. However, other dosage regimens may be useful and are not excluded herefrom. The desired dosage can be delivered by a single bolus administration, by multiple bolus administrations, or by continuous infusion administration of antibody, depending on the pattern of pharmacokinetic decay that the practitioner wishes to achieve.

These suggested amounts of antibody are subject to a great deal of therapeutic discretion. The key factor in selecting an appropriate dose and scheduling is the result obtained. Factors for consideration in this context include the particular disorder being treated, the particular mammal being treated, the clinical condition of the individual patient, the cause of the disorder, the site of delivery of the antibody, the particular type of antibody, the method of administration, the scheduling of administration, and other factors known to medical practitioners.

Therapeutic agents of the invention may be frozen or lyophilized for storage and reconstituted in a suitable sterile carrier prior to use. Lyophilization and reconstitution can lead to varying degrees of antibody activity loss. Dosages may have to be adjusted to compensate. Generally, pH between 6 and 8 is preferred.

### Therapeutic Use

Myostatin plays a role in muscle development and a number of related disorders or diseases (see, *e.g.,* U.S. patent application 2003/0074680 and 2003/0082181). In adults, myostatin mRNA is primarily detected in skeletal muscle although lower concentrations are also found in adipose tissue and cardiac tissue (Sharma, M., et al, J. Cell Physiol. 180:1, 1999). Myostatin knockout mice have two- to threefold greater muscle mass than their wild type littermates. The increased muscle mass is the result of fiber hypertrophy and hyperplasia (McPherron, A., et al. Nature 387:83-90, 1997 and Zhu, X. et al., FEBS Letters 474:71). In addition, the myostatin knockout mice accumulate less fat than their wild type littermates but otherwise appear normal and healthy. Myostatin has also been recently shown to be an important regulator of adipogenesis (Rebbapragada, A., et al., Mol. and Cell. Bio. 23:7230-7242, 2003). Additionally, bone structure and content has been recently studied in myostatin deficient mice (Hamrick M.W., et al., J. Orthopaedic Research 21:1025, 2003; Hamrick, M.W., et al., Calcif Tissue Int 71:63, 2002.

Therefore, a pharmaceutical composition comprising an anti-myostatin monoclonal antibody of the invention may be used to increase muscle mass, increase bone density, or may be useful for the treatment of conditions wherein the presence of myostatin causes or contributes to undesirable pathological effects or decrease of myostatin levels has a therapeutic benefit in mammals including, but not limited to, the conditions of: muscle wasting, frailty, age-related sarcopenia, osteoporosis, obesity, muscular dystrophy, myopathy, cachexia, sepsis, osteoarthritis, COPD, renal failure, liver failure, cardiac failure or disease, metabolic syndrome and Type II diabetes.

The use of an anti-myostatin monoclonal antibody of the present invention for treating or preventing of at least one of the aforementioned disorders in which myostatin activity is detrimental or which benefits for decreased levels of bioactive myostatin is contemplated herein. Additionally, the use of an anti-myostatin monoclonal antibody of the present invention for use in the manufacture of a medicament for the treatment of at least one of the aforementioned disorders is contemplated. ,

As used herein, the terms "treatment", "treating", and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse affect attributable to the disease. "Treatment", as used herein, includes administration of a compound of the present invention for treatment of a disease or condition in a mammal, particularly in a human, and includes: (a) preventing the disease from occurring in a subject which may be predisposed to the disease but has not yet been diagnosed as having it; (b) inhibiting the disease, *i.e*., arresting its development; and (c) relieving the disease, i.e., causing regression of the disease or disorder or alleviating symptoms or complications thereof. Dosage regimens may be adjusted to provide the optimum desired response (*e.g*., a therapeutic or prophylactic response). For example, a single bolus may be administered, several divided doses may be administered over time or the dose may be proportionally reduced or increased as indicated by the exigencies of the therapeutic situation.

The following examples are offered for illustrative purposes only, and are not intended to limit the scope of the present invention in any way.

### EXAMPLES

### Example 1: Anti-Myostatin Fab Synthesis

Clones of anti-myostatin Fabs are isolated from a Fab library created by immunizing C57BI/6 wild-type mice using Omniclonal^{™} antibody technology (Biosite^{®}, San Diego, CA). The mice are immunized with an immunogenic polypeptide with the amino acid sequence: ANYCSGESEFVFLQKYPHTHLVHQA (SEQ ID NO: 43). This sequence is identical to the sequence spanning amino acids 40-64 of the mature form of human myostatin (SEQ ID NO: 2) with the exception that the Cys residue at position 47 in wild-type human mature myostatin (underlined in SEQ ID NO: 43 above) is changed to a Ser residue to prevent carrier or hapten linkage to the peptide at this residue. To improve the immunogenicity of this peptide the carrier protein, keyhole limpet hemocyanin, and a helper T-cell peptide are conjugated to the immunogenic peptide according to standard methods. The HCVR and LCVR CDR and framework amino acid sequences disclosed herein (Tables 1 and 2; Fig. 4 and 5) are identified as the sequences of Fabs from the library which bind mature myostatin (*e.g*., SEQ ID NO: 2) and bind the immunogenic peptide and neutralize a myostatin activity. Representative nucleotide sequences encoding the LCVR and HCVR of the Fabs are listed below in Table 3.

### Table 3 - Representative Nucleotide Seq Encoding Fab LCVR and HCVR

LCVR (SEQ ID NO: 44) HCVR (SEQ ID NO: 45)

### Example 2: ELISA Assays

### A. Anti-myostatin Fabs preferentially bind mature myostatin

Mouse anti-myostatin Fabs of the present invention (See, Figs. 4 and 5) are tested in an ELISA assay, in which binding of the Fab to mature myostatin (dimeric form) coated at various concentrations on a 96-well plate is measured. Binding of the Fabs to GDF-11 is also tested.

Each well of two 96-well plates is coated with 70 µl recombinant mouse myostatin (R&D systems, Cat. #788-G8/CF, carrier-free, 1µg/ml in carbonate buffer, pH 9.6) or 70 µl recombinant human GDF-11 (Peprotech, Inc., Cat. # 120-11, carrier-free, 1(µg/ml in carbonate buffer, pH 9.6). The plates are incubated at 4°C overnight. The wells are aspirated and washed twice with washing buffer (20 mM Tris (hydroxymethyl) aminomethane, pH 7.4, 0.15 M NaCl, 0.1% Tween-20). The plates are blocked with 200 µl blocking buffer per well (5% Carnation Instant milk in the above washing buffer) for 5 hours.

Fabs to be tested are diluted into blocking buffer at 10 µg/ml, 2 µg/ml, 0.4 µg/ml, 0.08 µg/ml, and 0.016 µg/ml. Fifty microliters of each Fab solution is added to the GDF-8 and GDF-11 coated wells in duplicate. The plates are incubated for 1 hour at room temperature. The wells are then washed 3 times with washing buffer.

Peroxidase-conjugated secondary antibody (50 µl goat anti-mouse kappa HRP (Southern Biotech), diluted 1:2000 in blocking buffer) is added to each well and incubated for 1 hour at room temperature. The wells are then washed 3 times with washing buffer. Fifty microliters of chromogenic substrate (*i.e*., OPD substrate) is added to each well and allowed to develop at room temperature for 13 minutes. The reaction is stopped by adding 100 µl 1N HCl to each well. The absorbance of the wells is read at OD of 490 nm. The average absorbance from duplicate wells is determined.

These data demonstrate that Fabs 3, 5 and 7 of the invention (Figs 4 and 5) bind to plate-bound human mature myostatin and preferentially bind to myostatin when compared to GDF-11 binding.

### B. Anti-myostatin Fabs bind peptide immunogen of myostatin (I).

Mouse anti-myostatin Fabs 3, 5, 7, 8, 9, 10, 11, 12, 14 and 15 are tested in an ELISA assay, in which binding of the Fab to the polypeptide used for immunization of mice ("peptide immunogen") is measured. This polypeptide spans amino acids 40 to 64 of mature myostatin and has the amino acid sequence
ANYCSGESEFVFLQKYPHTHLVHQA (SEQ ID NO: 43) as described herein.

Each well of two 96-well plates is coated with 70 µl of the peptide immunogen used to generate the Fabs (2 µg/ml in carbonate buffer, pH 9.6). The plates are incubated in a dry oven at 37°C overnight with the lids removed. The wells are aspirated and washed twice with washing buffer (20 mM Tris (hydroxymethyl) aminomethane, pH 7.4, 0.15 M NaCl, 0.1% Tween-20). The plates are blocked with 200 µl blocking buffer per well (5% BioRad blotting grade milk in the above washing buffer) for 2.5 hours.

The Fabs are diluted into blocking buffer at 10 µg/ml, 2 µg/ml, 0.4 µg/ml, 0.08 µg/ml, and 0.016 µg/ml. A rat anti-myostatin monoclonal antibody (R&D Systems, catalog #MAB788, clone #84214) and a polyclonal anti-myostatin antibody (R&D Systems, catalog #AF788), are used as controls at the above concentrations and are also diluted in blocking buffer. Fifty microliters of each antibody solution is added to the peptide-coated wells in duplicate. The plates are incubated for 1.5 hours at room temperature. The wells are then washed 3 times with washing buffer.

Peroxidase-conjugated secondary antibody (50 µl goat anti-mouse kappa HRP (Southern Biotech) for Fabs, 50 µl mouse anti-rat (Jackson ImmunoResearch) for monoclonal, 50 µl rabbit anti-goat (Jackson ImmunoResearch) for polyclonal, all diluted 1:2000 in blocking buffer is added to each well and incubated for 1 hour at room temperature. The wells are then washed 3 times with washing buffer. Fifty microliters of chromogenic substrate (*i.e*., OPD substrate) is added to each well and allowed to develop at room temperature for 10 minutes. The reaction is stopped by adding 100 µl 1N HCl to each well. The absorbance of the wells is read at 490 nm. The average absorbance from duplicate wells is determined, and these values are listed in Table 4, below.

The Fabs bind to plate-bound peptide immunogen. The polyclonal antibody also binds the peptide immunogen, to a lesser extent than the Fabs. The R&D monoclonal antibody binding is at background levels, which is consistent with the R&D monoclonal antibody recognizing a different epitope than that encompassed by the immunogenic peptide.

### C. Anti-myostatin Fabs binding various members of TGF-β superfamily.

Mouse anti-myostatin Fabs 3, 5, 7 (see Figs 4 and 5), and a polyclonal anti-myostatin antibody (R&D Systems) are tested in an ELISA assay, in which binding of the Fabs and antibody to family members of the antigen (GDF-8/myostatin) coated on a plate is measured. Binding of the Fabs to the following panel of TGF-beta superfamily members is tested: GDF-8/myostatin (control), GDF-11, BMP-2, BMP-5, BMP-6, BMP-7, Activin A, Activin B, TGF-alpha, TGF-betal, and TGF-beta2. IGF-1 is also tested as a negative control.

Each well of a 96-well plate is coated with 70 µl of one of the growth factors listed above (10 µg/ml in carbonate buffer, pH 9.6) in duplicate. See Table 5 below for sources and catalog numbers. The plates are incubated at 4°C overnight. The wells are aspirated and washed twice with washing buffer (20 mM Tris (hydroxymethyl) aminomethane, pH 7.4, 0.15 M NaCl, 0.1 % Tween-20). The plates are blocked with 200ul blocking buffer per well (5% Carnation Instant milk in the above washing buffer) for 3 hours.

Antibodies are diluted into blocking buffer at 10 µg/ml. Fifty microliters of each antibody solution is added to the growth factor-coated wells. The plates are incubated for 1 hour at room temperature. The wells are then washed 3 times with washing buffer.

Peroxidase-conjugated secondary antibody (50 µl goat anti-mouse kappa HRP (Southern Biotech) for the Fabs, 50 µl rabbit anti-goat (Jackson ImmunoResearch) for the polyclonal, diluted 1:2000 in blocking buffer) is added to each well and incubated for 1 hour at room temperature. The wells are then washed 3 times with washing buffer. Fifty microliters of chromogenic substrate (i.e., OPD substrate) is added to each well and allowed to develop at room temperature for 10 minutes. The reaction is stopped by adding 100 µl 1N HCl to each well. The absorbance of the wells is read at 490 nm. The average absorbance from duplicate wells is determined, and these values are listed in Table 6, below.

These data demonstrate that Fabs 3, 5, and 7 bind preferentially to myostatin than to the other proteins tested. Little or no binding above background is detected for the Fabs to any of the other TGF-beta superfamily members under these conditions, with the exception of a very small amount of binding to GDF-11 by Fab 3. The R&D anti-myostatin polyclonal antibody, however, also binds to GDF-11 (see Table 6 below).

**Table 5**

| | Source company | catalog # |
|---|---|---|
| GDF-8 | R&D Systems | 788-G8/CF |
| GDF-11 | Peprotech, Inc. | 120-11 |
| IGF-1 | R&D Systems | 291-G1 |
| BMP-2 | R&D Systems | 355-BEC/CF |
| BMP-5 | R&D Systems | 615-BM |
| BMP-6 | R&D Systems | 507-BP |
| BMP-7 | R&D Systems | 354-BP |
| Activin A | R&D Systems | 338-AC |
| Activin B | R&D Systems | 659-AB |
| TGF-a | R&D Systems | 239-A |
| TGF-b1 | Peprotech, Inc. | 100-21R |
| TGF-b2 | Peprotech, Inc. | 100-35 |

**Table 6**

| | GDF-8 | GDF-11 | IGF-1 | BMP-2 | BMP-5 | BMP-6 |
|---|---|---|---|---|---|---|
| R&D poly | 2.941 | 2.621 | 0.045 | 0.0525 | 0.038 | 0.0745 |
| Fab 3 | 0.4195 | 0.0705 | 0.034 | 0.0345 | 0.034 | 0.0335 |
| Fab 5 | 0.202 | 0.034 | 0.035 | 0.032 | 0.033 | 0.0325 |
| Fab 7 | 0.358 | 0.04 | 0.034 | 0.0335 | 0.0345 | 0.035 |
| | | | | | | |

| | BMP-7 | Activin A | Activin B | TGF-a | TGF-b1 | TGF-b2 |
|---|---|---|---|---|---|---|
| R&D poly | 0.042 | 0.049 | 0.0485 | 0.0445 | 0.0425 | 0.0525 |
| Fab 3 | 0.033 | 0.033 | 0.032 | 0.033 | 0.034 | 0.034 |
| Fab 5 | 0.032 | 0.033 | 0.033 | 0.033 | 0.0325 | 0.034 |
| Fab 7 | 0.031 | 0.0325 | 0.031 | 0.0315 | 0.032 | 0.0345 |

### Example 3 Myostatin Neutralization Assay_

Ectodermal explants are removed from stage 8-9 blastula Xenopus embryos by standard procedures and cultured in 0.5X MBS (1X MBS: 88 mM NaCl, 1 mM KCl, 0.7 mM CaCl₂, 1 mM MgSO₄, 5 mM HEPES, 2.5 mM NaHCO₃, 1:1000 v/v gentamycin, 0.1% bovine serum albumin) with the addition of growth factor (GDF8 or GDF11) plus or indicated, for 18 hours at 18°C, by which time control embryos reach the early neurula stage (stage 15-16). Explants are photographed and the length of each explant is measured using an image analysis algorithm designed for animal cap quantitation. Explants not treated with either growth factor or Fab (controls), round into balls of epidermis. Myostatin and GDF-11 induce mesoderm in these ectodermal explants which causes the explants to elongate and form dumbbell-like structures. Antibodies or Fabs, when tested for neutralizing activity, are added to the culture medium containing myostatin for the entire length of the culture period and their ability to inhibit the growth factor-induced elongation movements is assessed. Myostatin is added to the explants at 25 ng/ml. Antibodies or Fabs are added at 20 µg/ml. Fab34 is a Fab generated to an irrelevant antigen. Commercially available anti-myostatin polyclonal antibody was also tested; this antibody is produced in goats immunized with purified mouse GDF8 and demonstrated by the manufacturer to neutralize the elongation of Xenopus animal caps elicited by 25 ng/ml of murine GDF8 when present at about 10-50 µg/ml (R&D Systems, Inc. Cat. #AF788). A commercially available monoclonal anti-mouse GDF8 antibody was tested, this antibody is demonstrated by the manufacturer to neutralize elongation of Xenopus animal caps elicited by 25 ng/ml of murine GDF8 when present at about 10-20 µg/ml (R&D Systems Cat. #MAB788). Note that the ELISA data of Example 2 herein show this R&D antibody to bind to a different region of myostatin than the Fabs of the present invention.

ImagePro (v4.5.1.22, from Media Cybernetics) is used for the image processing. A macro is written to automate the image processing. The macro processes the image and records length in units of bits. Alternative measuring methods may be used as known in the art. Fabs 3, 5, 7, 8, 9, 10, 11, 12, 14 and 15 are able to significantly neutralize GDF8 activity in the animal cap assay.

### Example 4: Affinity Measurement of Monoclonal Fabs

The affinity (K_{D}) and Kₒₙ and K_{off} rates of anti-myostatin Fabs 3, 5, 7, 8, 9, 10, 11, 12, 14, 15 of the present invention are measured using a BIAcore^{®} 2000 instrument containing a CM5 sensor chip. The BIAcore^{®} utilizes the optical properties of surface plasmon resonance to detect alterations in protein concentration of interacting molecules within a dextran biosensor matrix. Except where noted, all reagents and materials are purchased from BIAcore^{®} AB (Upsala, Sweden). All measurements are performed at 25°C. Samples containing rat or human myostatin are dissolved in HBS-EP buffer (150 mM sodium chloride, 3 mM EDTA, 0.005% (w/v) surfactant P-20, and 10 mM HEPES, pH 7.4). A capture antibody, goat anti-mouse Kappa (Southern Biotechnology, Inc), is immobilized onto flow cells using amine-coupling chemistry. Flow cells (1-4) are activated for 7 minutes with a 1:1 mixture of 0.1 M N-hydroxysuccinimide and 0.1 M 3-(N,N-dimethylamino)propyl-N-ethylcarbodiimide at a flow rate of 10 µl/min. Goat anti-mouse Kappa (30 µg/mL in 10mM sodium acetate, pH 4.5) is manually injected over all 4 flow cells at a flow rate of 10 µL/min. The surface density is monitored and additional goat anti-mouse Kappa is injected if needed to individual cell until all flow cells reach a surface density of 4500-5000 response units (RU). Surfaces are blocked with a 7 minute injection of 1 M ethanolamine-HCl, pH 8.5 (10 µL/min). To ensure complete removal of any noncovalently bound goat anti-mouse Kappa, 15 µL of 10mM glycine, pH 1.5 is injected twice. Running buffer used for kinetic experiments contained 10mM HEPES, pH 7.4, 150mM NaCl, 0.005% P20.

Collection of kinetic binding data is performed at maximum flow rate (100 µL/min) and a low surface density to minimize mass transport effects. Each analysis cycle consists of (i) capture of 300-350 RU of Fabs (BioSite) by injection of 5-10 µL of 5 µg/ml solution over flow cell 2, 3 and 4 for different Fabs at a flow rate of 10 µL/min., (ii) 200 µL injection (2 min) of human myostatin (concentration range of 50 nM to 1.56 nM in 2-fold dilution increments) over all 4 flow cells with flow cell 1 as the reference flow cell, (iii) 10 min dissociation (buffer flow), (iv) regeneration of goat anti-mouse Kappa surface with a 15 sec injection of 10 mM glycine, pH 1.5, (v) a 30 sec blank injection of running buffer, and (vi) a 2 min stabilization time before start of next cycle. Signal is monitored as flow cell 2 minus flow cell 1, flow cell 3 minus flow cell 1 and flow cell 4 minus flow cell 1. Samples and a buffer blank are injected in duplicate in a random order. Data are processed using BIAevaluation 3.1 software and data are fit to a 1:1 binding model in CLAMP global analysis software.

Fabs 3, 5, 7, 8, 9, 10, 11, 12, 14 and 15 have K_{D} values between 7 x 10⁻⁶ and 4.0 x 10⁻⁸.

### Example 5 In vivo Mouse Model of Musculoskeletal Efficacy

Male ICR mice (8 weeks old, Taconic NY) are castrated (gonadectomized, GDX) according to approved procedures and allowed to waste for ten weeks. Age-matched sham- operated (Sham) mice are also obtained. Sham-operated mice are operated in the same manner as the castrated ones except their testes are not removed. Animals arere housed in a temperature-controlled room (24°C) with a reversed 12 hour light/dark cycle and water and food are available *ad libitum.*

In order to demonstrate *in vivo* efficacy, compound of the present invention is administered every other week by subcutaneous injection to the castrated eighteen week old mice (body weight about 48-50g) and age-matched sham mice. Test compound is administered to the animals in Phosphate-Buffered Saline (PBS). The castrated mice treated only with isotype-matched IgG1 are used as a treatment negative control.

Test animals (12 mice of each group) are dosed over a 15 week time-frame subcutaneously, with, e.g., 60 mg/kg/2 wk of a compound of the present invention. At each dosing time point, the dose given is adjusted according to the body weight of each animal. The following measurements are recorded at the beginning and end of the study: body weight, body muscle mass by quantitative magnetic resonance (QMR, Echo Medical Systems, TX) analysis and body grip-strength (Columbus Instruments, OH).

After the 15-week treatment, as an indicator of muscle activity the wet weight of the skeletal muscle (quadriceps) in the test groups are determined and compared to the weights in the castrated, IgG-only control group. As an indicator of skeletal activity, the bone mass (bone mineral density, BMD, mg/cc) of the femoral bones from test animals are similarly compared to the bone mass of the femoral bones from the castrated, IgG-only group by microcomputed tomography (qCT) (Research M, Stratec) analysis. The anti-myostatin antibody comprising Fab 3 had anabolic effects on both muscle and bone under conditions described here.

## Claims

1. An anti-myostatin monoclonal antibody or a functional fragment thereof that specifically binds a polypeptide consisting of amino acids 40-64 of a mature form of human myostatin as shown in SEQ ID NO: 46:

2. The monoclonal antibody of claim 1 or a functional fragment thereof which is competitively inhibited from binding mature human myostatin by a monoclonal antibody comprising two polypeptides with sequences selected from:
(i) SEQ ID NOs: 3 and 12,
(ii) SEQ ID NOs: 4 and 13,
(iii) SEQ ID NOs: 3 and 14,
(iv) SEQ ID NOs: 5 and 12,
(v) SEQ ID NOs: 6 and 15,
(vi) SEQ NOs: 7 and 17,
(vii) SEQ ID NOs: 8 and 12,
(viii) SEQ ID NOs: 9 and 16,
(ix) SEQ ID NOs: 10 and 12, and
(x) SEQ ID NOs: 11 and 12.

3. The monoclonal antibody or a functional fragment thereof of claim 1 or claim 2 which binds myostatin with at least 20-fold greater affinity than with which it binds GDF-11.

4. The monoclonal antibody or a functional fragment thereof of any of claims 1 to 3 comprising a peptide at LCVR CDR1 with the sequence as shown in SEQ ID NO: 18, a peptide at LCVR CDR2 with the sequence as shown in SEQ ID NO: 23, a peptide at LCVR CDR3 with the sequence as shown in SEQ ID NO: 24, a peptide at HCVR CDR1 with the sequence as shown in SEQ ID NO: 29, a peptide at HCVR CDR2 with the sequence as shown in SEQ ID NO: 32 and a peptide at HCVR CDR3 with the sequence as shown in SEQ ID NO: 36.

5. The monoclonal antibody or a functional fragment thereof of any of claims 1 to 3 comprising a peptide at LCVR CDR1 with the sequence as shown in SEQ ID NO: 19, a peptide at LCVR CDR2 with the sequence as shown in SEQ ID NO: 23, a peptide at LCVR CDR3 with the sequence as shown in SEQ ID NO: 25, a peptide at HCVR CDR1 with the sequence as shown in SEQ ID NO: 30, a peptide at HCVR CDR2 with the sequence as shown in SEQ ID NO: 33 and a peptide at HCVR CDR3 with the sequence as shown in SEQ ID NO: 37.

6. The monoclonal antibody or a functional fragment thereof of any of claims 1 to 3 comprising a peptide at LCVR CDR1 with the sequence as shown in SEQ ID NO: 18, a peptide at LCVR CDR2 with the sequence as shown in SEQ ID NO: 23, a peptide at LCVR CDR3 with the sequence as shown in SEQ ID NO: 24, a peptide at HCVR CDR1 with the sequence as shown in SEQ ID NO: 31, a peptide at HCVR CDR2 with the sequence as shown in SEQ ID NO: 32 and a peptide at HCVR CDR3 with the sequence as shown in SEQ ID NO: 36.

7. The monoclonal antibody or a functional fragment thereof of any of claims 1 to 3 comprising a peptide at LCVR CDR1 with the sequence as shown in SEQ ID NO: 20, a peptide at LCVR CDR2 with the sequence as shown in SEQ ID NO: 23, a peptide at LCVR CDR3 with the sequence as shown in SEQ ID NO: 25, a peptide at HCVR CDR1 with the sequence as shown in SEQ ID NO: 29, a peptide at HCVR CDR2 with the sequence as shown in SEQ ID NO: 32 and a peptide at HCVR CDR3 with the sequence as shown in SEQ ID NO: 36.

8. The monoclonal antibody or a functional fragment thereof of any of claims 1 to 3 comprising a peptide at LCVR CDR1 with the sequence as shown in SEQ ID NO: 20, a peptide at LCVR CDR2 with the sequence as shown in SEQ ID NO: 23, a peptide at LCVR CDR3 with the sequence as shown in SEQ ID NO: 26, a peptide at HCVR CDR1 with the sequence as shown in SEQ ID NO: 30, a peptide at HCVR CDR2 with the sequence as shown in SEQ ID NO: 34 and a peptide at HCVR CDR3 with the sequence as shown in SEQ ID NO: 36.

9. The monoclonal antibody or a functional fragment thereof of any of claims 1 to 3 comprising a peptide at LCVR CDR1 with the sequence as shown in SEQ ID NO: 18, a peptide at LCVR CDR2 with the sequence as shown in SEQ ID NO: 23, a peptide at LCVR CDR3 with the sequence as shown in SEQ ID NO: 24, a peptide at HCVR CDR1 with the sequence as shown in SEQ ID NO: 29, a peptide at HCVR CDR2 with the sequence as shown in SEQ ID NO: 35 and a peptide at HCVR CDR3 with the sequence as shown in SEQ ID NO: 36.

10. The monoclonal antibody or a functional fragment thereof of any of claims 1 to 3 comprising a peptide at LCVR CDR1 with the sequence as shown in SEQ ID NO: 18, a peptide at LCVR CDR2 with the sequence as shown in SEQ ID NO: 23, a peptide at LCVR CDR3 with the sequence as shown in SEQ ID NO: 27, a peptide at HCVR CDR1 with the sequence as shown in SEQ ID NO: 29, a peptide at HCVR CDR2 with the sequence as shown in SEQ ID NO: 32 and a peptide at HCVR CDR3 with the sequence as shown in SEQ ID NO: 36.

11. The monoclonal antibody or a functional fragment thereof of any of claims 1 to 3 comprising a peptide at LCVR CDR1 with the sequence as shown in SEQ ID NO: 21, a peptide at LCVR CDR2 with the sequence as shown in SEQ ID NO: 23, a peptide at LCVR CDR3 with the sequence as shown in SEQ ID NO: 28, a peptide at HCVR CDR1 with the sequence as shown in SEQ ID NO: 29, a peptide at HCVR CDR2 with the sequence as shown in SEQ ID NO: 32 and a peptide at HCVR CDR3 with the sequence as shown in SEQ ID NO: 36.

12. The monoclonal antibody or a functional fragment thereof of any of claims 1 to 3 comprising a peptide at LCVR CDR1 with the sequence as shown in SEQ ID NO: 20, a peptide at LCVR CDR2 with the sequence as shown in SEQ ID NO: 23, a peptide at LCVR CDR3 with the sequence as shown in SEQ ID NO: 24, a peptide at HCVR CDR1 with the sequence as shown in SEQ ID NO: 29, a peptide at HCVR CDR2 with the sequence as shown in SEQ ID NO: 32 and a peptide at HCVR CDR3 with the sequence as shown in SEQ ID NO: 36.

13. The monoclonal antibody or a functional fragment thereof of any of claims 1 to 3 comprising a peptide at LCVR CDR1 with the sequence as shown in SEQ ID NO: 22, a peptide at LCVR CDR2 with the sequence as shown in SEQ ID NO: 23, a peptide at LCVR CDR3 with the sequence as shown in SEQ ID NO: 27, a peptide at HCVR CDR1 with the sequence as shown in SEQ ID NO: 29, a peptide at HCVR CDR2 with the sequence as shown in SEQ ID NO: 32 and a peptide at HCVR CDR3 with the sequence as shown in SEQ ID NO: 36.

14. The monoclonal antibody of any one of claims 1 to 13 wherein the monoclonal antibody is a full-length antibody, a substantially intact antibody, a Fab fragment, a F(ab')₂ fragment or a single chain Fv fragment.

15. The monoclonal antibody or a functional fragment thereof of any one of claims 1 to 13 wherein the monoclonal antibody is a chimeric antibody.

16. The monoclonal antibody or a functional fragment thereof of any one of claims 1 to 13 wherein the monoclonal antibody is a humanized antibody.

17. The monoclonal antibody or a functional fragment thereof of any one of claims 1 to 13 wherein the constant region present in the antibody originates from the genome of an animal selected from domestic animals, sports animals and food-source animals.

18. A pharmaceutical composition comprising the antibody or a functional fragment thereof of any one of claims 1 to 17 and a pharmaceutically acceptable carrier.

19. The monoclonal antibody or a functional fragment thereof of any of claims 1 to 17 for use as a medicament.

20. The monoclonal antibody or a functional fragment thereof of any one of claims 1 to 17 for use in the prevention or treatment of muscle wasting, myopathy, muscular dystrophy, muscle weakness, frailty, cachexia, renal failure or disease, liver failure or disease, cardiac failure or disease, type II diabetes, metabolic syndrome, osteoporosis, obesity or COPD in a human.

21. A monoclonal antibody or a functional fragment thereof of any one of claims 1 to 17 for use in increasing muscle mass in a mammal.

## Patentansprüche

1. Monoklonaler anti-Myostatin-Antikörper oder ein funktionelles Fragment hiervon, der (das) spezifisch ein Polypeptid bindet, das aus den Aminosäuren 40-64 einer reifen Form von humanem Myostatin, wie in der SEQ ID Nr: 46 gezeigt, besteht:

2. Monoklonaler Antikörper nach Anspruch 1 oder ein funktionelles Fragment hiervon, der (das) an einem Binden von reifem humanem Myostatin kompetitiv gehindert wird durch einen monoklonalen Antikörper, der zwei Polypeptide mit Sequenzen umfasst, die aus den folgenden ausgewählt sind:
(i) SEQ ID Nr: 3 und 12,
(ii) SEQ ID Nr: 4 und 13,
(iii) SEQ ID Nr: 3 und 14,
(iv) SEQ ID Nr: 5 und 12,
(v) SEQ ID Nr: 6 und 15,
(vi) SEQ ID Nr: 7 und 17,
(vii) SEQ ID Nr: 8 und 12,
(viii) i) SEQ ID Nr: 9 und 16,
(ix) SEQ ID Nr: 10 und 12 und
(x) SEQ ID Nr: 11 und 12.

3. Monoklonaler Antikörper oder ein funktionelles Fragment hiervon nach Anspruch 1 oder 2, der (das) Myostatin mit mindestens 20-fach höherer Affinität als mit der, mit der er (es) GDF-11 bindet, bindet.

4. Monoklonaler Antikörper oder ein funktionelles Fragment hiervon nach einem der Ansprüche 1 bis 3, umfassend ein Peptid an der LCVR-CDR1 mit der in SEQ ID Nr: 18 gezeigten Sequenz, ein Peptid an der LCVR-CDR2 mit der in SEQ ID Nr. 23 gezeigten Sequenz, ein Peptid an der LCVR-CDR3 mit der in SEQ ID Nr. 24 gezeigten Sequenz, ein Peptid an der HCVR-CDR1 mit der in SEQ ID Nr: 29 gezeigten Sequenz, ein Peptid an der HCVR-CDR2 mit der in SEQ ID Nr: 32 gezeigten Sequenz und ein Peptid an der HCVR-CDR3 mit der in SEQ ID Nr: 36 gezeigten Sequenz.

5. Monoklonaler Antikörper oder ein funktionelles Fragment hiervon nach einem der Ansprüche 1 bis 3, umfassend ein Peptid an der LCVR-CDR1 mit der in SEQ ID Nr: 19 gezeigten Sequenz, ein Peptid an der LCVR-CDR2 mit der in SEQ ID Nr. 23 gezeigten Sequenz, ein Peptid an der LCVR-CDR3 mit der in SEQ ID Nr. 25 gezeigten Sequenz, ein Peptid an der HCVR-CDR1 mit der in SEQ ID Nr: 30 gezeigten Sequenz, ein Peptid an der HCVR-CDR2 mit der in SEQ ID Nr: 33 gezeigten Sequenz und ein Peptid an der HCVR-CDR3 mit der in SEQ ID Nr: 37 gezeigten Sequenz.

6. Monoklonaler Antikörper oder ein funktionelles Fragment hiervon nach einem der Ansprüche 1 bis 3, umfassend ein Peptid an der LCVR-CDR1 mit der in SEQ ID Nr: 18 gezeigten Sequenz, ein Peptid an der LCVR-CDR2 mit der in SEQ ID Nr. 23 gezeigten Sequenz, ein Peptid an der LCVR-CDR3 mit der in SEQ ID Nr. 24 gezeigten Sequenz, ein Peptid an der HCVR-CDR1 mit der in SEQ ID Nr: 31 gezeigten Sequenz, ein Peptid an der HCVR-CDR2 mit der in SEQ ID Nr: 32 gezeigten Sequenz und ein Peptid an der HCVR-CDR3 mit der in SEQ ID Nr: 36 gezeigten Sequenz.

7. Monoklonaler Antikörper oder ein funktionelles Fragment hiervon nach einem der Ansprüche 1 bis 3, umfassend ein Peptid an der LCVR-CDR1 mit der in SEQ ID Nr: 20 gezeigten Sequenz, ein Peptid an der LCVR-CDR2 mit der in SEQ ID Nr. 23 gezeigten Sequenz, ein Peptid an der LCVR-CDR3 mit der in SEQ ID Nr. 25 gezeigten Sequenz, ein Peptid an der HCVR-CDR1 mit der in SEQ ID Nr: 29 gezeigten Sequenz, ein Peptid an der HCVR-CDR2 mit der in SEQ ID Nr: 32 gezeigten Sequenz und ein Peptid an der HCVR-CDR3 mit der in SEQ ID Nr: 36 gezeigten Sequenz.

8. Monoklonaler Antikörper oder ein funktionelles Fragment hiervon nach einem der Ansprüche 1 bis 3, umfassend ein Peptid an der LCVR-CDR1 mit der in SEQ ID Nr: 20 gezeigten Sequenz, ein Peptid an der LCVR-CDR2 mit der in SEQ ID Nr. 23 gezeigten Sequenz, ein Peptid an der LCVR-CDR3 mit der in SEQ ID Nr. 26 gezeigten Sequenz, ein Peptid an der HCVR-CDR1 mit der in SEQ ID Nr: 30 gezeigten Sequenz, ein Peptid an der HCVR-CDR2 mit der in SEQ ID Nr: 34 gezeigten Sequenz und ein Peptid an der HCVR-CDR3 mit der in SEQ ID Nr: 36 gezeigten Sequenz.

9. Monoklonaler Antikörper oder ein funktionelles Fragment hiervon nach einem der Ansprüche 1 bis 3, umfassend ein Peptid an der LCVR-CDR1 mit der in SEQ ID Nr: 18 gezeigten Sequenz, ein Peptid an der LCVR-CDR2 mit der in SEQ ID Nr. 23 gezeigten Sequenz, ein Peptid an der LCVR-CDR3 mit der in SEQ ID Nr. 24 gezeigten Sequenz, ein Peptid an der HCVR-CDR1 mit der in SEQ ID Nr: 29 gezeigten Sequenz, ein Peptid an der HCVR-CDR2 mit der in SEQ ID Nr: 35 gezeigten Sequenz und ein Peptid an der HCVR-CDR3 mit der in SEQ ID Nr: 36 gezeigten Sequenz.

10. Monoklonaler Antikörper oder ein funktionelles Fragment hiervon nach einem der Ansprüche 1 bis 3, umfassend ein Peptid an der LCVR-CDR1 mit der in SEQ ID Nr: 18 gezeigten Sequenz, ein Peptid an der LCVR-CDR2 mit der in SEQ ID Nr. 23 gezeigten Sequenz, ein Peptid an der LCVR-CDR3 mit der in SEQ ID Nr. 27 gezeigten Sequenz, ein Peptid an der HCVR-CDR1 mit der in SEQ ID Nr: 29 gezeigten Sequenz, ein Peptid an der HCVR-CDR2 mit der in SEQ ID Nr: 32 gezeigten Sequenz und ein Peptid an der HCVR-CDR3 mit der in SEQ ID Nr: 36 gezeigten Sequenz.

11. Monoklonaler Antikörper oder ein funktionelles Fragment hiervon nach einem der Ansprüche 1 bis 3, umfassend ein Peptid an der LCVR-CDR1 mit der in SEQ ID Nr: 21 gezeigten Sequenz, ein Peptid an der LCVR-CDR2 mit der in SEQ ID Nr. 23 gezeigten Sequenz, ein Peptid an der LCVR-CDR3 mit der in SEQ ID Nr. 28 gezeigten Sequenz, ein Peptid an der HCVR-CDR1 mit der in SEQ ID Nr: 29 gezeigten Sequenz, ein Peptid an der HCVR-CDR2 mit der in SEQ ID Nr: 32 gezeigten Sequenz und ein Peptid an der HCVR-CDR3 mit der in SEQ ID Nr: 36 gezeigten Sequenz.

12. Monoklonaler Antikörper oder ein funktionelles Fragment hiervon nach einem der Ansprüche 1 bis 3, umfassend ein Peptid an der LCVR-CDR1 mit der in SEQ ID Nr: 20 gezeigten Sequenz, ein Peptid an der LCVR-CDR2 mit der in SEQ ID Nr. 23 gezeigten Sequenz, ein Peptid an der LCVR-CDR3 mit der in SEQ ID Nr. 24 gezeigten Sequenz, ein Peptid an der HCVR-CDR1 mit der in SEQ ID Nr: 29 gezeigten Sequenz, ein Peptid an der HCVR-CDR2 mit der in SEQ ID Nr: 32 gezeigten Sequenz und ein Peptid an der HCVR-CDR3 mit der in SEQ ID Nr: 36 gezeigten Sequenz.

13. Monoklonaler Antikörper oder ein funktionelles Fragment hiervon nach einem der Ansprüche 1 bis 3, umfassend ein Peptid an der LCVR-CDR1 mit der in SEQ ID Nr: 22 gezeigten Sequenz, ein Peptid an der LCVR-CDR2 mit der in SEQ ID Nr. 23 gezeigten Sequenz, ein Peptid an der LCVR-CDR3 mit der in SEQ ID Nr. 27 gezeigten Sequenz, ein Peptid an der HCVR-CDR1 mit der in SEQ ID Nr: 29 gezeigten Sequenz, ein Peptid an der HCVR-CDR2 mit der in SEQ ID Nr: 32 gezeigten Sequenz und ein Peptid an der HCVR-CDR3 mit der in SEQ ID Nr: 36 gezeigten Sequenz.

14. Monoklonaler Antikörper nach einem der Ansprüche 1 bis 13, wobei der monoklonale Antikörper ein eine volle Länge aufweisender Antikörper, ein im Wesentlichen intakter Antikörper, ein Fab-Fragment, ein F(ab')₂-Fragment oder ein Einzelketten-Fv-Fragment ist.

15. Monoklonaler Antikörper oder ein funktionelles Fragment hiervon nach einem der Ansprüche 1 bis 13, wobei der monoklonale Antikörper ein chimerer Antikörper ist.

16. Monoklonaler Antikörper oder ein funktionelles Fragment hiervon nach einem der Ansprüche 1 bis 13, wobei der monoklonale Antikörper ein humanisierter Antikörper ist.

17. Monoklonaler Antikörper oder ein funktionelles Fragment hiervon nach einem der Ansprüche 1 bis 13, wobei die in dem Antikörper vorhandene konstante Region aus den Genom eines Tieres stammt, das aus Haustieren, Sporttieren und als Nahrungsquelle dienenden Tieren ausgewählt ist.

18. Pharmazeutische Zusammensetzung, die den Antikörper oder ein funktionelles Fragment hiervon nach einem der Ansprüche 1 bis 17 und einen pharmazeutisch akzeptablen Träger umfasst.

19. Monoklonaler Antikörper oder ein funktionelles Fragment hiervon nach einem der Ansprüche 1 bis 17 zur Verwendung als ein Medikament.

20. Monoklonaler Antikörper oder ein funktionelles Fragment hiervon nach einem der Ansprüche 1 bis 17 zur Verwendung bei der Prävention oder Behandlung von Muskelschwund, Myopathie, muskulärer Dystrophie, Muskelschwäche, Gebrechlichkeit, Cachexie, Niereninsuffizienz oder -erkrankung, Leberinsuffizienz oder -erkrankung, Herzinsuffizienz oder -erkrankung, Typ-II-Diabetes, metabolischem Syndrom, Osteoporose, Fettleibigkeit oder COPD bei einem Menschen.

21. Monoklonaler Antikörper oder ein funktionelles Fragment hiervon nach einem der Ansprüche 1 bis 17 zur Verwendung bei einer Steigerung der Muskelmasse bei einem Säuger.

## Revendications

1. Anticorps monoclonal dirigé contre la myostatine, ou un de ses fragments fonctionnels, qui se lie de manière spécifique à un polypeptide constitué par les acides aminés 40-64 d'une forme mature de la myostatine humaine comme représenté dans la SEQ ID NO: 46 :

2. Anticorps monoclonal selon la revendication 1 ou un de ses fragments fonctionnels dont la liaison à la myostatine humaine mature est soumise à une inhibition compétitive par un anticorps monoclonal comprenant deux polypeptides dont les séquences sont choisies parmi :
(i) les SEQ ID NO: 3 et 12 ;
(ii) les SEQ ID NO: 4 et 13 ;
(iii) les SEQ ID NO: 3 et 14 ;
(iv) les SEQ ID NO: 5 et 12 ;
(v) les SEQ ID NO: 6 et 15 ;
(vi) les SEQ ID NO: 7 et 17 ;
(vii) les SEQ ID NO: 8 et 12 ;
(viii) les SEQ ID NO: 9 et 16 ;
(ix) les SEQ ID NO: 10 et 12 ; et
(x) les SEQ ID NO: 11 et 12.

3. Anticorps monoclonal ou un de ses fragments fonctionnels selon la revendication 1 ou 2, qui se lie à la myostatine avec une affinité au moins 20 fois supérieure à l'affinité avec laquelle il se lie au GDF-11.

4. Anticorps monoclonal ou un de ses fragments fonctionnels selon l'une quelconque des revendications 1 à 3, comprenant un peptide à LCVR CDR1 possédant la séquence telle que représentée dans la SEQ ID NO: 18, un peptide à LCVR CDR2 possédant la séquence telle que représentée dans la SEQ ID NO: 23, un peptide à LCVR CDR3 possédant la séquence telle que représentée dans la SEQ ID NO: 24, un peptide à HCVR CDR1 possédant la séquence telle que représentée dans la SEQ ID NO: 29, un peptide à HCVR CDR2 possédant la séquence telle que représentée dans la SEQ ID NO: 32, et un peptide à HCVR CDR3 possédant la séquence telle que représentée dans la SEQ ID NO: 36.

5. Anticorps monoclonal ou un de ses fragments fonctionnels selon l'une quelconque des revendications 1 à 3, comprenant un peptide à LCVR CDR1 possédant la séquence telle que représentée dans la SEQ ID NO: 19, un peptide à LCVR CDR2 possédant la séquence telle que représentée dans la SEQ ID NO: 23, un peptide à LCVR CDR3 possédant la séquence telle que représentée dans la SEQ ID NO: 25, un peptide à HCVR CDR1 possédant la séquence telle que représentée dans la SEQ ID NO: 30, un peptide à HCVR CDR2 possédant la séquence telle que représentée dans la SEQ ID NO: 33, et un peptide à HCVR CDR3 possédant la séquence telle que représentée dans la SEQ ID NO: 37.

6. Anticorps monoclonal ou un de ses fragments fonctionnels selon l'une quelconque des revendications 1 à 3, comprenant un peptide à LCVR CDR1 possédant la séquence telle que représentée dans la SEQ ID NO: 18, un peptide à LCVR CDR2 possédant la séquence telle que représentée dans la SEQ ID NO: 23, un peptide à LCVR CDR3 possédant la séquence telle que représentée dans la SEQ ID NO: 24, un peptide à HCVR CDR1 possédant la séquence telle que représentée dans la SEQ ID NO: 31, un peptide à HCVR CDR2 possédant la séquence telle que représentée dans la SEQ ID NO: 32, et un peptide à HCVR CDR3 possédant la séquence telle que représentée dans la SEQ ID NO: 36.

7. Anticorps monoclonal ou un de ses fragments fonctionnels selon l'une quelconque des revendications 1 à 3, comprenant un peptide à LCVR CDR1 possédant la séquence telle que représentée dans la SEQ ID NO: 20, un peptide à LCVR CDR2 possédant la séquence telle que représentée dans la SEQ ID NO: 23, un peptide à LCVR CDR3 possédant la séquence telle que représentée dans la SEQ ID NO: 25, un peptide à HCVR CDR1 possédant la séquence telle que représentée dans la SEQ ID NO: 29, un peptide à HCVR CDR2 possédant la séquence telle que représentée dans la SEQ ID NO: 32, et un peptide à HCVR CDR3 possédant la séquence telle que représentée dans la SEQ ID NO: 36.

8. Anticorps monoclonal ou un de ses fragments fonctionnels selon l'une quelconque des revendications 1 à 3, comprenant un peptide à LCVR CDR1 possédant la séquence telle que représentée dans la SEQ ID NO: 20, un peptide à LCVR CDR2 possédant la séquence telle que représentée dans la SEQ ID NO: 23, un peptide à LCVR CDR3 possédant la séquence telle que représentée dans la SEQ ID NO: 26, un peptide à HCVR CDR1 possédant la séquence telle que représentée dans la SEQ ID NO: 30, un peptide à HCVR CDR2 possédant la séquence telle que représentée dans la SEQ ID NO: 34, et un peptide à HCVR CDR3 possédant la séquence telle que représentée dans la SEQ ID NO: 36.

9. Anticorps monoclonal ou un de ses fragments fonctionnels selon l'une quelconque des revendications 1 à 3, comprenant un peptide à LCVR CDR1 possédant la séquence telle que représentée dans la SEQ ID NO: 18, un peptide à LCVR CDR2 possédant la séquence telle que représentée dans la SEQ ID NO: 23, un peptide à LCVR CDR3 possédant la séquence telle que représentée dans la SEQ ID NO: 24, un peptide à HCVR CDR1 possédant la séquence telle que représentée dans la SEQ ID NO: 29, un peptide à HCVR CDR2 possédant la séquence telle que représentée dans la SEQ ID NO: 35, et un peptide à HCVR CDR3 possédant la séquence telle que représentée dans la SEQ ID NO: 36.

10. Anticorps monoclonal ou un de ses fragments fonctionnels selon l'une quelconque des revendications 1 à 3, comprenant un peptide à LCVR CDR1 possédant la séquence telle que représentée dans la SEQ ID NO: 18, un peptide à LCVR CDR2 possédant la séquence telle que représentée dans la SEQ ID NO: 23, un peptide à LCVR CDR3 possédant la séquence telle que représentée dans la SEQ ID NO: 27, un peptide à HCVR CDR1 possédant la séquence telle que représentée dans la SEQ ID NO: 29, un peptide à HCVR CDR2 possédant la séquence telle que représentée dans la SEQ ID NO: 32, et un peptide à HCVR CDR3 possédant la séquence telle que représentée dans la SEQ ID NO: 36.

11. Anticorps monoclonal ou un de ses fragments fonctionnels selon l'une quelconque des revendications 1 à 3, comprenant un peptide à LCVR CDR1 possédant la séquence telle que représentée dans la SEQ ID NO: 21, un peptide à LCVR CDR2 possédant la séquence telle que représentée dans la SEQ ID NO: 23, un peptide à LCVR CDR3 possédant la séquence telle que représentée dans la SEQ ID NO: 28, un peptide à HCVR CDR1 possédant la séquence telle que représentée dans la SEQ ID NO: 29, un peptide à HCVR CDR2 possédant la séquence telle que représentée dans la SEQ ID NO: 32, et un peptide à HCVR CDR3 possédant la séquence telle que représentée dans la SEQ ID NO: 36.

12. Anticorps monoclonal ou un de ses fragments fonctionnels selon l'une quelconque des revendications 1 à 3, comprenant un peptide à LCVR CDR1 possédant la séquence telle que représentée dans la SEQ ID NO: 20, un peptide à LCVR CDR2 possédant la séquence telle que représentée dans la SEQ ID NO: 23, un peptide à LCVR CDR3 possédant la séquence telle que représentée dans la SEQ ID NO: 24, un peptide à HCVR CDR1 possédant la séquence telle que représentée dans la SEQ ID NO: 29, un peptide à HCVR CDR2 possédant la séquence telle que représentée dans la SEQ ID NO: 32, et un peptide à HCVR CDR3 possédant la séquence telle que représentée dans la SEQ ID NO: 36.

13. Anticorps monoclonal ou un de ses fragments fonctionnels selon l'une quelconque des revendications 1 à 3, comprenant un peptide à LCVR CDR1 possédant la séquence telle que représentée dans la SEQ ID NO: 22, un peptide à LCVR CDR2 possédant la séquence telle que représentée dans la SEQ ID NO: 23, un peptide à LCVR CDR3 possédant la séquence telle que représentée dans la SEQ ID NO: 27, un peptide à HCVR CDR1 possédant la séquence telle que représentée dans la SEQ ID NO: 29, un peptide à HCVR CDR2 possédant la séquence telle que représentée dans la SEQ ID NO: 32, et un peptide à HCVR CDR3 possédant la séquence telle que représentée dans la SEQ ID NO: 36.

14. Anticorps monoclonal selon l'une quelconque des revendications 1 à 13, dans lequel l'anticorps monoclonal est un anticorps de longueur totale, un anticorps essentiellement intact, un fragment Fab, un fragment F(ab')₂, ou un fragment Fv monocaténaire.

15. Anticorps monoclonal ou un de ses fragments fonctionnels selon l'une quelconque des revendications 1 à 13, dans lequel l'anticorps monoclonal est un anticorps chimère.

16. Anticorps monoclonal ou un de ses fragments fonctionnels selon l'une quelconque des revendications 1 à 13, dans lequel l'anticorps monoclonal est un anticorps humanisé.

17. Anticorps monoclonal ou un de ses fragments fonctionnels selon l'une quelconque des revendications 1 à 13, dans lequel la région constante présente dans l'anticorps provient du génome d'un animal choisi parmi des animaux domestiques, des animaux destinés à des événements sportifs et des animaux utilisés comme source alimentaire.

18. Composition pharmaceutique comprenant l'anticorps ou un de ses fragments fonctionnels selon l'une quelconque des revendications 1 à 17, et un support pharmaceutiquement acceptable.

19. Anticorps monoclonal ou un de ses fragments fonctionnels selon l'une quelconque des revendications 1 à 17, à utiliser comme médicament.

20. Anticorps monoclonal ou un de ses fragments fonctionnels selon l'une quelconque des revendications 1 à 17, à utiliser dans la prévention ou le traitement de la maigreur musculaire, de la myopathie, de la dystrophie musculaire, de la faiblesse musculaire, de la fragilité, de la cachexie, de l'insuffisance rénale ou d'une néphropathie, de l'insuffisance hépatique ou d'un trouble hépatique, de l'insuffisance cardiaque ou d'une cardiopathie, du diabète de type II, du syndrome métabolique, de l'ostéoporose, de l'obésité ou de la COPD chez un être humain.

21. Anticorps monoclonal ou un de ses fragments fonctionnels selon l'une quelconque des revendications 1 à 17, à utiliser pour augmenter la masse musculaire dans un mammifère.


### SEQUENCE LISTING

<110> Eli Lilly and company
<120> Anti-Myostatin Antibodies
<130> X-16397
<140> US 60/559,621
   <141> 2004-04-05
<150> US 60/555,456
   <151> 2004-03-24
<160> 56
<170> PatentIn version 3.3
<210> 1
   <211> 375
   <212> PRT
   <213> Homo sapiens
<400> 1
   Met Gln Lys Leu Gln Leu Cys Val Tyr Ile Tyr Leu Phe Met Leu Ile
   1 5 10 15
   Val Ala Gly Pro Val Asp Leu Asn Glu Asn Ser Glu Gln Lys Glu Asn
   20 25 30
   Val Glu Lys Glu Gly Leu Cys Asn Ala Cys Thr Trp Arg Gln Asn Thr
   35 40 45
   Lys Ser Ser Arg Ile Glu Ala Ile Lys Ile Gln Ile Leu Ser Lys Leu
   50 55 60
   Arg Leu Glu Thr Ala Pro Asn Ile Ser Lys Asp Val Ile Arg Gln Leu
   65 70 75 80
   Leu Pro Lys Ala Pro Pro Leu Arg Glu Leu Ile Asp Gln Tyr Asp Val
   85 90 95
   Gln Arg Asp Asp Ser Ser Asp Gly Ser Leu Glu Asp Asp Asp Tyr His
   100 105 110
   Ala Thr Thr Glu Thr Ile Ile Thr Met Pro Thr Glu Ser Asp Phe Leu
   115 120 125
   Met Gln Val Asp Gly Lys Pro Lys Cys Cys Phe Phe Lys Phe Ser Ser
   130 135 140
   Lys Ile Gln Tyr Asn Lys Val Val Lys Ala Gln Leu Trp Ile Tyr Leu
   145 150 155 160
   Arg Pro Val Glu Thr Pro Thr Thr Val Phe Val Gln Ile Leu Arg Leu
   165 170 175
   Ile Lys Pro Met Lys Asp Gly Thr Arg Tyr Thr Gly Ile Arg Ser Leu
   180 185 190
   Lys Leu Asp Met Asn Pro Gly Thr Gly Ile Trp Gln Ser Ile Asp Val
   195 200 205
   Lys Thr Val Leu Gln Asn Trp Leu Lys Gln Pro Glu Ser Asn Leu Gly
   210 215 220
   Ile Glu Ile Lys Ala Leu Asp Glu Asn Gly His Asp Leu Ala Val Thr
   225 230 235 240
   Phe Pro Gly Pro Gly Glu Asp Gly Leu Asn Pro Phe Leu Glu Val Lys
   245 250 255
   Val Thr Asp Thr Pro Lys Arg Ser Arg Arg Asp Phe Gly Leu Asp Cys
   260 265 270
   Asp Glu His Ser Thr Glu Ser Arg Cys Cys Arg Tyr Pro Leu Thr Val
   275 280 285
   Asp Phe Glu Ala Phe Gly Trp Asp Trp Ile Ile Ala Pro Lys Arg Tyr
   290 295 300
   Lys Ala Asn Tyr Cys Ser Gly Glu Cys Glu Phe Val Phe Leu Gln Lys
   305 310 315 320
   Tyr Pro His Thr His Leu Val His Gln Ala Asn Pro Arg Gly Ser Ala
   325 330 335
   Gly Pro Cys Cys Thr Pro Thr Lys Met Ser Pro Ile Asn Met Leu Tyr
   340 345 350
   Phe Asn Gly Lys Glu Gln Ile Ile Tyr Gly Lys Ile Pro Ala Met Val
   355 360 365
   Val Asp Arg Cys Gly Cys Ser
   370 375
<210> 2
   <211> 109
   <212> PRT
   <213> Homo sapiens
<400> 2
   Asp Phe Gly Leu Asp Cys Asp Glu His Ser Thr Glu Ser Arg Cys Cys
   1 5 10 15
   Arg Tyr Pro Leu Thr Val Asp Phe Glu Ala Phe Gly Trp Asp Trp Ile
   20 25 30
   Ile Ala Pro Lys Arg Tyr Lys Ala Asn Tyr Cys Ser Gly Glu Cys Glu
   35 40 45
   Phe Val Phe Leu Gln Lys Tyr Pro His Thr His Leu Val His Gln Ala
   50 55 60
   Asn Pro Arg Gly Ser Ala Gly Pro Cys Cys Thr Pro Thr Lys Met Ser
   65 70 75 80
   Pro Ile Asn Met Leu Tyr Phe Asn Gly Lys Glu Gln Ile Ile Tyr Gly
   85 90 95
   Lys Ile Pro Ala Met Val Val Asp Arg Cys Gly Cys Ser
   100 105
<210> 3
   <211> 109
   <212> PRT
   <213> Mus sp.
<400> 3
   Gln Ile Val Leu Thr Gln Ser Pro Ala Ile Met Ser Ala Ser Pro Gly
   1 5 10 15
   Glu Lys Val Thr Met Thr Cys Ser Ala Ser Ser Ser Ile Ser Tyr Met
   20 25 30
   His Trp Tyr Gln Gln Lys Pro Gly Thr Ser Pro Lys Arg Trp Ile Tyr
   35 40 45
   Asp Thr Ser Lys Leu Ala Ser Gly Val Pro Ala Arg Phe Ser Gly Ser
   50 55 60
   Gly Ser Gly Thr Ser Tyr Ser Leu Thr Ile Ser Ser Met Glu Ala Glu
   65 70 75 80
   Asp Ala Ala Thr Tyr Tyr Cys Gln Gln Trp Tyr Ser Asn Pro Leu Thr
   85 90 95
   Phe Gly Ala Gly Thr Lys Leu Glu Leu Lys Arg Ala Asp
   100 105
<210> 4
   <211> 109
   <212> PRT
   <213> Mus sp.
<400> 4
   Gln Val Val Leu Thr Gln Ser Pro Ala Ile Met Ser Ala Ser Leu Gly
   1               5                   10                  15
   Glu Lys Val Thr Met Thr Cys Ser Ala Ser Ser Ser Val His Tyr Met
               20                  25                  30
   His Trp Tyr Gln Gln Lys Ser Gly Thr Ser Pro Lys Arg Trp Ile Tyr
           35                  40                  45
   Asp Thr Ser Lys Leu Ala Ser Gly Val Pro Ala Arg Phe Ser Gly Ser
       50                  55                  60
   Gly Ser Gly Thr Ser Tyr Ser Leu Thr Ile Ser Ser Met Glu Ala Glu
   65                  70                  75                  80
   Asp Ala Ala Thr Tyr Tyr Cys Gln Gln Trp Ser Ser Asn Pro Leu Thr
                   85                  90                  95
   Phe Gly Ala Gly Thr Lys Leu Glu Leu Lys Arg Ala Asp
               100                 105
<210> 5
   <211> 109
   <212> PRT
   <213> Mus sp.
<400> 5
   Gln Ile Val Leu Thr Gln Ser Pro Ala Ile Met Ser Ala Ser Pro Gly
   1               5                   10                  15
   Glu Lys Val Thr Met Thr Cys Ser Ala Ser Ser Ser Val Ser Tyr Met
               20                  25                  30
   His Trp Tyr Gln Gln Lys Ser Gly Thr Ser Pro Lys Arg Trp Ile Tyr
           35                  40                  45
   Asp Thr Ser Lys Leu Ala Ser Gly Val Pro Ala Arg Phe Ser Gly Ser
       50                  55                  60
   Gly Ser Gly Thr Ser Tyr Ser Leu Thr Ile Ser Ser Met Glu Ala Glu
   65                  70                  75                  80
   Asp Ala Ala Thr Tyr Tyr Cys Gln Gln Trp Ser Ser Asn Pro Leu Thr
                   85                  90                  95
   Phe Gly Ala Gly Thr Lys Leu Glu Leu Lys Arg Ala Asp
   100 105
<210> 6
   <211> 109
   <212> PRT
   <213> Mus sp.
<400> 6
   Gln Ile Val Leu Thr Gln Ser Pro Ala Ile Met Ser Ala Ser Pro Gly
   1 5 10 15
   Glu Lys Val Thr Met Thr Cys Ser Ala Ser Ser Ser Val Ser Tyr Met
   20 25 30
   His Trp Tyr Gln Gln Lys Ser Gly Thr Ser Pro Lys Arg Trp Ile Tyr
   35 40 45
   Asp Thr Ser Lys Leu Ala Ser Gly Val Pro Val Arg Phe Ser Gly Ser
   50 55 60
   Gly Ser Gly Thr Ser Tyr Ser Leu Thr Ile Ser Ser Met Glu Ala Glu
   65 70 75 80
   Asp Ala Ala Thr Tyr Tyr Cys Gln Gln Trp Ser Arg Asn Pro Leu Thr
   85 90 95
   Phe Gly Ala Gly Thr Lys Leu Glu Leu Lys Arg Ala Asp
   100 105
<210> 7
   <211> 109
   <212> PRT
   <213> Mus sp.
<400> 7
   Gln Val Val Leu Thr Gln Ser Pro Ala Ile Met Ser Ala Ser Pro Gly
   1 5 10 15
   Glu Lys Val Thr Met Thr Cys Ser Ala Ser Ser Ser Ile Ser Tyr Met
   20 25 30
   His Trp Tyr Gln Gln Lys Pro Gly Thr Ser Pro Lys Arg Trp Ile Tyr
   35 40 45
   Asp Thr Ser Lys Leu Ala Ser Gly Val Pro Ala Arg Phe Ser Gly Ser
   50 55 60
   Gly Ser Gly Thr Ser Tyr Ser Leu Thr Ile Ser Ser Met Glu Ala Glu
   65 70 75 80
   Asp Ala Ala Thr Tyr Tyr Cys Gln Gln Trp Tyr Ser Asn Pro Leu Thr
   85 90 95
   Phe Gly Ala Gly Thr Lys Leu Glu Leu Lys Arg Ala Asp
   100 105
<210> 8
   <211> 109
   <212> PRT
   <213> Mus sp.
<400> 8
   Gln Ile Val Leu Thr Gln Ser Pro Ala Ile Met Ser Ala Ser Pro Gly
   1 5 10 15
   Glu Lys Val Thr Met Thr Cys Ser Ala Ser Ser Ser Ile Ser Tyr Met
   20 25 30
   His Trp Tyr Gln Gln Lys Pro Gly Thr Ser Pro Lys Arg Trp Ile Tyr
   35 40 45
   Asp Thr Ser Lys Leu Ala Ser Gly Val Pro Ala Arg Phe Ser Gly Ser
   50 55 60
   Gly Ser Gly Thr Ser Tyr Ser Leu Thr Ile Ser Ser Met Glu Ala Glu
   65 70 75 80
   Asp Ala Ala Thr Tyr Tyr Cys Gln Gln Trp Asn Ser Asn Pro Leu Thr
   85 90 95
   Phe Gly Ala Gly Thr Lys Leu Glu Leu Lys Arg Ala Asp
   100 105
<210> 9
   <211> 109
   <212> PRT
   <213> Mus sp.
<400> 9
   Gln Val Val Leu Thr Gln Ser Pro Ala Ile Met Ser Ala Ser Pro Gly
   1 5 10 15
   Glu Lys Val Thr Met Thr Cys Ser Ala Ser Ser Ser Val Tyr Tyr Met
   20 25 30
   His Trp Tyr Gln Gln Arg Ser Gly Ala Ser Pro Lys Arg Trp Ile Tyr
   35 40 45
   Asp Thr Ser Lys Leu Ala Ser Gly Val Pro Ala Arg Phe Ser Gly Ser
   50 55 60
   Gly Ser Gly Thr Ser Tyr Ser Leu Thr Ile Ser Ser Met Glu Ala Glu
   65 70 75 80
   Asp Ala Ala Thr Tyr Tyr Cys Gln Gln Trp Thr Tyr Asn Pro Leu Thr
   85 90 95
   Phe Gly Ala Gly Thr Lys Leu Glu Leu Lys Arg Ala Asp
   100 105
<210> 10
   <211> 109
   <212> PRT
   <213> Mus sp.
<400> 10
   Gln Ile Val Leu Thr Gln Ser Pro Ala Ile Met Ser Ala Ser Pro Gly
   1 5 10 15
   Glu Lys Val Thr Met Thr Cys Ser Ala Ser Ser Ser Val Ser Tyr Met
   20 25 30
   His Trp Tyr Gln Gln Lys Pro Gly Thr Ser Pro Lys Arg Trp Ile Tyr
   35 40 45
   Asp Thr Ser Lys Leu Ala Ser Gly Val Pro Ala Arg Phe Ser Gly Ser
   50 55 60
   Gly Ser Gly Thr Ser Tyr Ser Leu Thr Ile Ser Ser Met Glu Ala Glu
   65 70 75 80
   Asp Ala Ala Thr Tyr Tyr Cys Gln Gln Trp Tyr Ser Asn Pro Leu Thr
   85 90 95
   Phe Gly Ala Gly Thr Lys Leu Glu Leu Lys Arg Ala Asp
   100 105
<210> 11
   <211> 109
   <212> PRT
   <213> Mus sp.
<400> 11
   Gln Ile Val Leu Thr Gln Ser Pro Ala Ile Met Ser Ala Ser Pro Gly
   1 5 10 15
   Glu Glu Val Thr Met Thr Cys Ser Ala Ser Ser Ser Ile Asn Tyr Met
   20 25 30
   His Trp Tyr Gln Gln Lys Ser Gly Thr Ser Pro Lys Arg Trp Ile Tyr
   35 40 45
   Asp Thr Ser Lys Leu Ala Ser Gly Val Pro Ala Arg Phe Ser Gly Ser
   50 55 60
   Gly Ser Gly Thr Ser Tyr Ser Leu Thr Ile Ser Ser Met Glu Ala Glu
   65 70 75 80
   Asp Ala Ala Thr Tyr Tyr Cys Gln Gln Trp Asn Ser Asn Pro Leu Thr
   85 90 95
   Phe Gly Gly Gly Thr Lys Leu Glu Leu Lys Arg Ala Asp
   100 105
<210> 12
   <211> 124
   <212> PRT
   <213> Mus sp.
<400> 12
   Gln Val Thr Leu Lys Glu Ser Gly Pro Gly Ile Leu Gln Ser Ser Gln
   1               5                   10                  15
   Thr Leu Ser Leu Thr Cys Ser Leu Ser Gly Phe Ser Leu Arg Thr Ser
               20                  25                  30
   Gly Met Ser Val Ser Trp Ile Arg Gln Ser Ser Gly Lys Gly Leu Glu
           35                  40                  45
   Trp Leu Ala His Ile Tyr Trp Asp Asp Asp Lys Arg Tyr Asn Pro Ser
       50                  55                  60
   Leu Arg Asn Arg Leu Thr Ile Ser Lys Asp Thr Leu Arg Asn Gln Val
   65                  70                  75                  80
   Phe Leu Lys Ile Thr Ser Val Gly Thr Ala Asp Thr Ala Thr Tyr Tyr
                   85                  90                  95
   Cys Ala Arg Arg Ala Ile Thr Thr Val Ile Gly Gly Gly Thr Met Asp
               100                 105                 110
   Tyr Trp Gly Gln Gly Thr Ser Val Thr Val Ser Ser
           115                 120
<210> 13
   <211> 124
   <212> PRT
   <213> Mus sp.
<400> 13
   Gln Val Thr Leu Lys Glu Ser Gly Pro Gly Ile Leu Gln Ser Ser Gln
   1 5 10 15
   Thr Leu Ser Leu Thr Cys Ser Phe Ser Gly Phe Ser Leu Ser Thr Ser
   20 25 30
   Gly Met Ser Val Ser Trp Ile Arg Gln Ser Ser Gly Lys Gly Leu Glu
   35 40 45
   Trp Leu Ala His Ile Tyr Trp Asp Asp Asp Lys Arg Tyr Asn Pro Ser
   50 55 60
   Leu Arg Ser Arg Leu Thr Ile Ser Lys Asp Thr Ser Arg Asn Gln Val
   65 70 75 80
   Phe Leu Lys Ile Thr Ser Val Asp Thr Ala Asp Thr Ala Thr Tyr Tyr
   85 90 95
   Cys Ala Arg Arg Gly Ile Thr Thr Val Leu Gly Gly Gly Thr Met Asp
   100 105 110
   Tyr Trp Gly Gln Gly Thr Ser Val Thr Val Ser Ser
   115 120
<210> 14
   <211> 123
   <212> PRT
   <213> Mus sp.
<400> 14
   Gln Val Thr Leu Lys Ser Gly Pro Gly Ile Leu Gln Ser Ser Gln Thr
   1 5 10 15
   Leu Thr Leu Thr Cys Ser Leu Ser Gly Phe Ser Leu Thr Thr Ser Gly
   20 25 30
   Met Ile Val Ser Trp Ile Arg Gln Ser Ser Gly Arg Gly Leu Glu Trp
   35 40 45
   Leu Ala His Ile Tyr Trp Asp Asp Asp Lys Arg Tyr Asn Pro Ser Leu
   50 55 60
   Arg Asn Arg Leu Thr Ile Ser Lys Asp Thr Leu Arg Asn Gln Val Phe
   65 70 75 80
   Leu Trp Ile Ser Ser Val Gly Thr Ala Asp Thr Ala Thr Tyr Tyr Cys
   85 90 95
   Ala Arg Arg Ala Ile Thr Thr Val Ile Gly Gly Gly Thr Met Asp Tyr
   100 105 110
   Trp Gly Gln Gly Thr Ser Val Thr Val Ser Ser
   115 120
<210> 15
   <211> 124
   <212> PRT
   <213> Mus sp.
<400> 15
   Gln Val Thr Leu Lys Glu Ser Gly Pro Gly Ile Leu Gln Ser Ser Gln
   1 5 10 15
   Thr Leu Ser Leu Thr Cys Ser Val Ser Gly Phe Ser Leu Ser Thr Ser
   20 25 30
   Gly Met Ser Val Ser Trp Ile Arg Gln Pro Ser Gly Lys Gly Leu Glu
   35 40 45
   Trp Leu Ala His Ile Tyr Trp Asp Asp Asp Lys Arg Tyr Asn Pro Ser
   50 55 60
   Leu Lys Ser Arg Leu Thr Ile Ser Lys Asp Thr Ser Arg Asn Gln Val
   65 70 75 80
   Phe Leu Lys Ile Thr Ser Val Asp Thr Ala Asp Thr Ala Thr Tyr Tyr
   85 90 95
   Cys Ala Arg Arg Ala Ile Thr Thr Val Leu Gly Gly Gly Thr Met Asp
   100 105 110
   Tyr Trp Gly Gln Gly Thr Ser Val Thr Val Ser Ser
   115 120
<210> 16
   <211> 124
   <212> PRT
   <213> Mus sp.
<400> 16
   Gln Val Thr Leu Lys Glu Ser Gly Pro Gly Met Leu Gln Ser Ser Gln
   1 5 10 15
   Thr Leu Ser Leu Thr Cys Ser Leu Ser Gly Phe Ser Leu Arg Thr Ser
   20 25 30
   Gly Met Ser Val Ser Trp Ile Arg Gln Ser Ser Gly Lys Gly Leu Glu
   35 40 45
   Trp Leu Ala His Ile Tyr Trp Asp Asp Asp Lys Arg Tyr Asn Pro Ser
   50 55 60
   Leu Arg Asn Arg Leu Thr Ile Ser Lys Asp Thr Leu Arg Asn Gln Val
   65 70 75 80
   Phe Leu Lys Ile Thr Ser Val Gly Thr Ala Asp Thr Ala Thr Tyr Tyr
   85 90 95
   Cys Ala Arg Arg Ala Ile Thr Thr Val Ile Gly Gly Gly Thr Met Asp
   100 105 110
   Tyr Trp Gly Gln Gly Thr Ser Val Thr Val Ser Ser
   115 120
<210> 17
   <211> 124
   <212> PRT
   <213> Mus sp.
<400> 17
   Gln Val Thr Leu Lys Glu Ser Gly Pro Gly Ile Leu Gln Pro Ser Gln
   1 5 10 15
   Thr Leu Ser Leu Thr Cys Ser Leu Ser Gly Phe Ser Leu Arg Thr Ser
   20 25 30
   Gly Met Ser Val Ser Trp Ile Arg Gln Ser Ser Gly Lys Gly Leu Glu
   35 40 45
   Trp Leu Ala His Ile Tyr Trp Asp Asp Asp Glu Arg Tyr Asn Pro Ser
   50 55 60
   Leu Arg Asn Arg Leu Thr Ile Ser Lys Asp Thr Leu Arg Asn Gln Val
   65 70 75 80
   Phe Leu Lys Ile Thr Ser Val Gly Thr Ala Asp Thr Ala Thr Tyr Tyr
   85 90 95
   Cys Ala Arg Arg Ala Ile Thr Thr Val Ile Gly Gly Gly Thr Met Asp
   100 105 110
   Tyr Trp Gly Gln Gly Thr Ser Val Thr Val Ser Ser
   115 120
<210> 18
   <211> 10
   <212> PRT
   <213> Mus sp.
<400> 18
   Ser Ala Ser Ser Ser Ile Ser Tyr Met His
   1 5 10
<210> 19
   <211> 10
   <212> PRT
   <213> Mus sp.
<400> 19
   Ser Ala Ser Ser Ser Val His Tyr Met His
   1 5 10
<210> 20
   <211> 10
   <212> PRT
   <213> Mus sp.
<400> 20
   Ser Ala Ser Ser Ser Val Ser Tyr Met His
   1 5 10
<210> 21
   <211> 10
   <212> PRT
   <213> Mus sp.
<400> 21
   Ser Ala Ser Ser Ser Val Tyr Tyr Met His
   1 5 10
<210> 22
   <211> 10
   <212> PRT
   <213> Mus sp.
<400> 22
   Ser Ala Ser Ser Ser Ile Asn Tyr Met His
   1 5 10
<210> 23
   <211> 7
   <212> PRT
   <213> Mus sp.
<400> 23
   Asp Thr Ser Lys Leu Ala Ser
   1 5
<210> 24
   <211> 9
   <212> PRT
   <213> Mus sp.
<400> 24
   Gln Gln Trp Tyr Ser Asn Pro Leu Thr
   1 5
<210> 25
   <211> 9
   <212> PRT
   <213> Mus sp.
<400> 25
   Gln Gln Trp Ser Ser Asn Pro Leu Thr
   1 5
<210> 26
   <211> 9
   <212> PRT
   <213> Mus sp.
<400> 26
   Gln Gln Trp Ser Arg Asn Pro Leu Thr
   1 5
<210> 27
   <211> 9
   <212> PRT
   <213> Mus sp.
<400> 27
   Gln Gln Trp Asn Ser Asn Pro Leu Thr
   1 5
<210> 28
   <211> 9
   <212> PRT
   <213> Mus sp.
<400> 28
   Gln Gln Trp Thr Tyr Asn Pro Leu Thr
   1 5
<210> 29
   <211> 12
   <212> PRT
   <213> Mus sp.
<400> 29
   Gly Phe Ser Leu Arg Thr Ser Gly Met Ser Val Ser
   1 5 10
<210> 30
   <211> 12
   <212> PRT
   <213> Mus sp.
<400> 30
   Gly Phe Ser Leu Ser Thr Ser Gly Met Ser Val Ser
   1 5 10
<210> 31
   <211> 12
   <212> PRT
   <213> Mus sp.
<400> 31
   Gly Phe Ser Leu Thr Thr Ser Gly Met Ile Val Ser
   1 5 10
<210> 32
   <211> 16
   <212> PRT
   <213> Mus sp.
<400> 32
   His Ile Tyr Trp Asp Asp Asp Lys Arg Tyr Asn Pro Ser Leu Arg Asn
   1 5 10 15
<210> 33
   <211> 16
   <212> PRT
   <213> Mus sp.
<400> 33
   His Ile Tyr Trp Asp Asp Asp Lys Arg Tyr Asn Pro Ser Leu Arg Ser
   1 5 10 15
<210> 34
   <211> 16
   <212> PRT
   <213> Mus sp.
<400> 34
   His Ile Tyr Trp Asp Asp Asp Lys Arg Tyr Asn Pro Ser Leu Lys Ser
   1 5 10 15
<210> 35
   <211> 16
   <212> PRT
   <213> Mus sp.
<400> 35
   His Ile Tyr Trp Asp Asp Asp Glu Arg Tyr Asn Pro Ser Leu Arg Asn
   1 5 10 15
<210> 36
   <211> 14
   <212> PRT
   <213> Mus sp.
<400> 36
   Arg Ala Ile Thr Thr Val Ile Gly Gly Gly Thr Met Asp Tyr
   1 5 10
<210> 37
   <211> 14
   <212> PRT
   <213> Mus sp.
<400> 37
   Arg Gly Ile Thr Thr Val Leu Gly Gly Gly Thr Met Asp Tyr
   1 5 10
<210> 38
   <211> 10
   <212> PRT
   <213> Mus sp.
<220>
   <221> MISC_FEATURE
   <222> (6)..(6)
   <223> X is a hydrophobic amino acid
<220>
   <221> MISC_FEATURE
   <222> (7)..(7)
   <223> X is Ser, Thr, His, Tyr or Asn
<400> 38
   Ser Ala Ser Ser Ser Xaa Xaa Tyr Met His
   1               5                   10
<210> 39
   <211> 109
   <212> PRT
   <213> Homo sapiens
<220>
   <221> MISC_FEATURE
   <222> (1)..(1)
   <223> X is Asp or Asn
<220>
   <221> MISC_FEATURE
   <222> (2)..(2)
   <223> X is Phe or Leu
<220>
   <221> MISC_FEATURE
   <222> (11)..(11)
   <223> X is Thr or Ser
<220>
   <221> MISC_FEATURE
   <222> (46)..(46)
   <223> X is Glu or Gln
<220>
   <221> MISC_FEATURE
   <222> (49)..(49)
   <223> X is Phe or Tyr
<220>
   <221> MISC_FEATURE
   <222> (50)..(50)
   <223> X is Val or Met
<220>
   <221> MISC_FEATURE
   <222> (52)..(52)
   <223> X is Leu or Met
<220>
   <221> MISC_FEATURE
   <222> (62)..(62)
   <223> X is His or Gln
<220>
   <221> MISC_FEATURE
   <222> (89)..(89)
   <223> X is Gly or Asp
<220>
   <221> MISC_FEATURE
   <222> (91)..(91)
   <223> X is Glu or Gln
<220>
   <221> MISC_FEATURE
   <222> (100)..(100)
   <223> X is Ala or Gly
<400> 39
   Xaa Xaa Gly Leu Asp Cys Asp Glu His Ser Xaa Glu Ser Arg Cys Cys
   1               5                   10                  15
   Arg Tyr Pro Leu Thr Val Asp Phe Glu Ala Phe Gly Trp Asp Trp Ile
               20                  25                  30
   Ile Ala Pro Lys Arg Tyr Lys Ala Asn Tyr Cys Ser Gly Xaa Cys Glu
           35                  40                  45
   Xaa Xaa Phe Xaa Gln Lys Tyr Pro His Thr His Leu Val Xaa Gln Ala
       50                  55                  60
   Asn Pro Arg Gly Ser Ala Gly Pro Cys Cys Thr Pro Thr Lys Met Ser
   65                  70                  75                  80
   Pro Ile Asn Met Leu Tyr Phe Asn Xaa Lys Xaa Gln Ile Ile Tyr Gly
                   85                  90                  95
   Lys Ile Pro Xaa Met Val Val Asp Arg Cys Gly Cys Ser
               100                 105
<210> 40
   <211> 109
   <212> PRT
   <213> Homo sapiens
<400> 40
   Asn Leu Gly Leu Asp Cys Asp Glu His Ser Ser Glu Ser Arg Cys Cys
   1 5 10 15
   Arg Tyr Pro Leu Thr Val Asp Phe Glu Ala Phe Gly Trp Asp Trp Ile
   20 25 30
   Ile Ala Pro Lys Arg Tyr Lys Ala Asn Tyr Cys Ser Gly Gln Cys Glu
   35 40 45
   Tyr Met Phe Met Gln Lys Tyr Pro His Thr His Leu Val Gln Gln Ala
   50 55 60
   Asn Pro Arg Gly Ser Ala Gly Pro Cys Cys Thr Pro Thr Lys Met Ser
   65 70 75 80
   Pro Ile Asn Met Leu Tyr Phe Asn Asp Lys Gln Gln Ile Ile Tyr Gly
   85 90 95
   Lys Ile Pro Gly Met Val Val Asp Arg Cys Gly Cys Ser
   100 105
<210> 41
   <211> 16
   <212> PRT
   <213> Mus sp.
<220>
   <221> MISC_FEATURE
   <222> (8)..(8)
   <223> X is Lys, Arg, Glu or Asp
<220>
   <221> MISC_FEATURE
   <222> (15)..(15)
   <223> X is Lys or Arg
<220>
   <221> MISC_FEATURE
   <222> (16)..(16)
   <223> X is Ser, Thr, Asn or Gln
<400> 41
   His Ile Tyr Trp Asp Asp Asp Xaa Arg Tyr Asn Pro Ser Leu Xaa Xaa
   1 5 10 15
<210> 42
   <211> 14
   <212> PRT
   <213> Mus sp.
<220>
   <221> MISC_FEATURE
   <222> (2)..(2)
   <223> X is Ala or Gly
<220>
   <221> MISC_FEATURE
   <222> (7)..(7)
   <223> X is Ile, Leu or Val
<400> 42
   Arg Xaa Ile Thr Thr Val Xaa Gly Gly Gly Thr Met Asp Tyr
   1               5                   10
<210> 43
   <211> 25
   <212> PRT
   <213> Mus sp.
<400> 43
   Ala Asn Tyr Cys Ser Gly Glu Ser Glu Phe Val Phe Leu Gln Lys Tyr
   1               5                   10                  15
   Pro His Thr His Leu Val His Gln Ala
               20                  25
<210> 44
   <211> 327
   <212> DNA
   <213> Mus sp.
<400> 44
   caaattgttc tcacccagtc tccagcaatc atgtctgcat ctccagggga gaaggtcacc     60
   atgacctgca gtgccagctc aagtataagt tacatgcact ggtaccagca gaagccaggc    120
   acctccccca aaagatggat ttatgacaca tccaaactgg cttctggagt ccctgctcgc    180
   ttcagtggca gtgggtctgg gacctcttac tctctcacaa tcagcagcat ggaggctgaa    240
   gatgctgcca cttattactg ccagcagtgg tatagtaacc cactcacgtt cggtgctggg    300
   accaagctgg agctgaaacg ggctgat                                        327
<210> 45
   <211> 372
   <212> DNA
   <213> Mus sp.
<400> 45
   caggttacgc tgaaagagtc tggccctggg atattgcagt cctcccagac cctcagtctg    60
   acttgttctc tctctgggtt ttcactgaga acgtctggta tgagtgtgag ctggattcgt   120
   cagtcttcag gaaagggtct ggagtggctg gcacacattt attgggatga tgacaagcgc   180
   tataacccat ccctgaggaa ccgactcaca atctccaagg ataccttgag aaaccaggtc   240
   ttcctcaaga tcaccagtgt gggcactgca gatactgcca catactactg tgctcgaaga   300
   gctattacta cggtaatagg gggagggact atggactact ggggtcaagg aacctcagtc   360
   accgtctcct ca                                                       372
<210> 46
   <211> 25
   <212> PRT
   <213> Mus sp.
<400> 46
   Ala Asn Tyr Cys Ser Gly Glu Cys Glu Phe Val Phe Leu Gln Lys Tyr
   1               5                   10                  15
   Pro His Thr His Leu Val His Gln Ala
               20                  25
<210> 47
   <211> 12
   <212> PRT
   <213> Mus sp.
<400> 47
   Gly Phe Ser Leu Arg Thr Ser Gly Ser Ser Val Ser
   1               5                   10
<210> 48
   <211> 12
   <212> PRT
   <213> Mus sp.
<400> 48
   Gly Phe Ser Leu Arg Lys Ser Gly Met Ser Val Ser
   1               5                   10
<210> 49
   <211> 12
   <212> PRT
   <213> Mus sp.
<400> 49
   Gly Phe Ser Leu Arg Thr Val Gly Met Ser Val Ser
   1 5 10
<210> 50
   <211> 12
   <212> PRT
   <213> Mus sp.
<400> 50
   Gly Phe Ser Leu Arg Thr Leu Gly Met Ser Val Ser
   1 5 10
<210> 51
   <211> 12
   <212> PRT
   <213> Mus sp.
<400> 51
   Gly Phe Ser Leu Arg Thr Leu Gly Ser Ser Val Ser
   1 5 10
<210> 52
   <211> 12
   <212> PRT
   <213> Mus sp.
<400> 52
   Gly Phe Ser Leu Arg Lys Val Gly Ser Ser Val Ser
   1 5 10
<210> 53
   <211> 12
   <212> PRT
   <213> Mus sp.
<400> 53
   Gly Phe Ser Leu Arg Lys Leu Gly Ser Ser Val Ser
   1 5 10
<210> 54
   <211> 12
   <212> PRT
   <213> Mus sp.
<400> 54
   Gly Phe Ser Leu Arg Lys Ser Gly Ser Ser Val Ser
   1 5 10
<210> 55
   <211> 12
   <212> PRT
   <213> Mus sp.
<220>
   <221> MISC_FEATURE
   <222> (5)..(5)
   <223> X is Arg, Lys, Thr or Ser
<220>
   <221> MISC_FEATURE
   <222> (6)..(6)
   <223> X is Thr or Lys
<220>
   <221> MISC_FEATURE
   <222> (7)..(7)
   <223> X is ser, Val or Leu
<220>
   <221> MISC_FEATURE
   <222> (9)..(9) or Ser
   <223> X is Met or ser
<220>
   <221> MISC_FEATURE
   <222> (10)..(10)
   <223> X is ser, Thr, Ile, Leu or Val
<400> 55
   Gly Phe Ser Leu Xaa Xaa Xaa Gly Xaa Xaa Val Ser
   1               5                   10
<210> 56
   <211> 9
   <212> PRT
   <213> Mus sp.
<220>
   <221> MISC_FEATURE
   <222> (4)..(4)
   <223> X is Tyr, ser, Asn or Thr
<220>
   <221> MISC_FEATURE
   <222> (5)..(5)
   <223> X is Arg, Lys, Tyr, ser or Thr
<400> 56
   Gln Gln Trp Xaa Xaa Asn Pro Leu Thr
   1               5